# EUROPÄISCHE PATENTSCHRIFT

(11) **EP 2 222 393 B1**
(45) Veröffentlichungstag und Bekanntmachung des Hinweises auf die Patenterteilung: **10.09.2014**
(21) Anmeldenummer: 08863547.9
(22) Anmeldetag: 19.12.2008
(51) Int. Cl.: B01F 17/34

(54) **VERFAHREN ZUR HERSTELLUNG VON ÖL IN WASSER EMULSIONEN AUS SELBSTEMULGIERENDEN GELKONZENTRATEN**
METHOD FOR PRODUCING OIL-IN-WATER EMULSIONS FROM SELF-EMULSIFYING GEL CONCENTRATES
PROCÉDÉ DE FABRICATION D'ÉMULSIONS HUILE DANS L'EAU À PARTIR DE CONCENTRÉS DE GELS AUTO-ÉMULSIFIANTS

(30) Priorität: 24.12.2007 DE 102007063134
(43) Veröffentlichungstag der Anmeldung: 01.09.2010
(73) Patentinhaber: SASOL Germany GmbH, 20537 Hamburg (DE)
(72) Erfinder: KWETKAT, Klaus, 59192 Bergkamen (DE); DAHMS, Gerd, W., 47138 Duisburg (DE); JAKOBS, Britta, 40764 Langenfeld (DE)
(74) Vertreter: Müller Schupfner & Partner Patent- und Rechtsanwaltspartnerschaft mbB
(86) Internationale Anmeldenummer: PCT/DE2008/002115
(87) Internationale Veröffentlichungsnummer: WO 2009/080005

(56) Entgegenhaltungen:
- EP-A- 1 524 029
- DE-A1- 10 346 515
- DE-A1-102004 031 550
- ADLER-NISSEN ET AL: "Apparatus for Emulsion Production in Small Scale and Under Controlled Shear Conditions" FOOD AND BIOPRODUCTS PROCESSING, INSTITUTION OF CHEMICAL ENGINEERS, RUGBY, GB, Bd. 82, Nr. 4, 1. Dezember 2004 (2004-12-01), Seiten 311-319, XP022525519 ISSN: 0960-3085
- FISCHER ET AL: "Emulsion drops in external flow fields - The role of liquid interfaces" CURRENT OPINION IN COLLOID AND INTERFACE SCIENCE, LONDON, GB, Bd. 12, Nr. 4-5, 1. Oktober 2007 (2007-10-01), Seiten 196-205, XP022266051 ISSN: 1359-0294
- OSTROVSKY M V ET AL: "Mechanism of microemulsion formation in systems with low interfacial tension: Occurrence, properties, and behavior of microemulsions" JOURNAL OF COLLOID AND INTERFACE SCIENCE, ACADEMIC PRESS, NEW YORK, NY, US, Bd. 102, Nr. 1, 1. November 1984 (1984-11-01), Seiten 206-226, XP024206552 ISSN: 0021-9797 [gefunden am 1984-11-01]

## Beschreibung

Die vorliegende Erfindung betrifft ein Verfahren zur Herstellung von Öl in Wasser (O/W) Emulsionen aus selbstemulgierenden O/W Gelkonzentraten ohne Agitation, wie z.B. Rühren, oder im laminaren Strömungsfeld.

Emulsionen sind disperse Mehrphasensysteme aus mindestens zwei ineinander nahezu unlöslichen Flüssigkeiten. Im einfachsten Fall handelt es sich um ein Zweiphasensystem aus einer hydrophilen bzw. polaren, z.B. wässrigen, Phase und einer lipophilen bzw. apolaren öligen Phase. Die innere oder disperse Phase liegt in Form von Tröpfchen in der äußeren kontinuierlichen Phase vor. Je nach Art der inneren Phase unterscheidet man zwischen Öl in Wasser - Emulsionen, in denen die Ölphase die disperse Phase ist, und Wasser in Öl (W/O)- Emulsionen, in denen die Wasserphase die disperse Phase ist. Emulsionen enthalten neben der dispersen und der kontinuierlichen Phase auch Hilfsstoffe, die den Tropfenaufbruch erleichtern und die aufgebrochenen Tröpfchen gegen Koaleszenz stabilisieren.

Der Begriff der Mikroemulsion ist in der gängigen Literatur nicht einheitlich gebraucht Er beschreibt einphasige Systeme, bikontinuierliche Emulsionen, geschwollene Mizellen und andere Strukturen, die klar wie auch trübe sein können. Die Klassifikation einer Emulsion nur über die mittlere Tröpfchengröße als Mikroemulsion ist nicht möglich. Einigkeit herrscht aber, dass Mikroemulsionen thermodynamisch stabile Systeme sind, während andere Emulsionen im Unterschied hierzu kinetisch stabilisiert sind und im thermodynamischen Sinne als instabil bezeichnet werden müssen. Vorliegend wird daher ausschließlich auf die thermodynamische Definition zurückgegriffen.

Nanoemulsionen im Sinne der Erfindung sind Emulsionen mit mittleren Tröpfchengrößen kleiner ein Mikrometer. Emulsionen mit mittleren Tröpfchengrößen gleich oder größer ein Mikrometer sind im Sinne dieser Erfindung Makroemulsionen.

Emulsionen mit ausreichend kleiner Tröpfchengröße werden heute zumeist im turbulenten Strömungsfeld, das zumeist über Rotor-Stator Systeme oder unter Zuhilfenahme von Hochdruckhomogenisatoren oder und dies ist noch eher selten durch Ultraschall hergestellt, unter Ausnutzung der dadurch erzeugten Kavitationskräfte.

Unter "selbstemulgierenden" O/W Zusammensetzungen werden solche Zusammensetzungen verstanden, welche in Wasser spontan, d.h. ohne den Eintrag mechanischer Energie, wie Rühren oder gar Homogenisieren, zu einer O/W Emulsion mit diskreten Tröpfchen zerfallen, die in der Emulsion mobil sind.

Ein einfacher Test um festzustellen, ob die Zusammensetzung selbstemulgierend bei Raumtemperatur, d.h. bei 15 bis 30°C, insbesondere 20 bis 25°C, im Sinne der vorliegenden Erfindung ist, ist es 5 ml bis 10 ml der Zusammensetzung auf 90 bis 95 ml deionisiertes Wasser aufzubringen. Wenn - auch ohne Durchmischen - eine sich nicht entmischende, ggf. milchige, O/W Emulsion resultiert, die für zumindest eine Stunde stabil ist, handelt es sich um eine selbstemulgierende Zusammensetzung im Sinne der vorliegenden Erfindung.

Es sind bereits Systeme für die Herstellung von O/W-Emulsionen beschrieben, die als selbstemulgierend bezeichnet werden. Jedoch wird häufig eine sehr unterschiedliche Auffassung darüber vertreten, was selbstemulgierend ist. In vielen Fällen ist es auch bei vermeintlich selbstemulgierenden bzw. als selbstemulgierend bezeichneten Systemen noch notwendig, heftig zu rühren bzw. zumindest die Komponenten durch Schütteln durchzumischen.

Es gibt z.B. flüssige Vorkonzentrate, typischerweise Öle - in der Regel wasserfrei - welche öllösliche Emulgatoren enthalten, und oft in Kombination mit Hydrokolloiden und Elektrolyten eingesetzt werden. Aus derartigen Vorkonzentraten resultierende O/W-Emulsionen sind häufig nur von durchschnittlicher Qualität hinsichtlich der Lagerstabilität. Für die Selbstemulgierbarkeit der Vorkonzentrate ist die Löslichkeit des Emulgatorsystems in der Ölphase von entscheidender Bedeutung. Feine Öltröpfchen sind hierbei häufig nur herstellbar, wenn die Ölphase und die wasserlöslichen Komponenten spezifisch ausgewählt und in ihren Konzentrationen aufeinander abgestimmt sind. Hierdurch verlieren die Systeme aber an Flexibilität, weil die Konzentrate nicht mehr ausreichend vielseitig eingesetzt werden können.

Andere selbstemulgierende Systeme beruhen auf O/W Emulsionen, die mit Öl aufgefüllt selbstemulgierende Systeme ergeben. Hier ist die Flexibilität sehr viel größer, notwendigerweise muss aber überwiegend Öl im Konzentrat vorhanden sein, was zu einer deutlichen Einschränkung des Nutzers dieser Konzentrate führt. Die Herstellung der Emulsionskonzentrate erfordert nach wie vor den Einsatz von Homogenisierungswerkzeugen und somit hohen Schergradienten und hoher Turbulenz.

DE 10346515 A1 beschreibt ein Verfahren zur Herstellung von Öl-in-Wasser- und Wasser-in-Öl-Emulsionen, bei denen ein Emulgatorkonzentrat, welches maximal 20 Gew.% Wasser enthält, in einer flüssigen Ölphase vorgelegt wird. Die Wasserphase wird in die emulgatorhaltige Ölphase unter Rühren eingetragen und emulgiert. Nach der DE 10346515 A1 werden zur Herstellung von Nanoemulsionen Alkyloligoglucosid und Polyglycerinpolyhydroxystearat, die öllöslichen Bestandteile des Emulgatorkonzentrates, in der Ölphase vorgelegt. Der wasserlösliche Bestandteil Acylglutamat wird in der Wasserphase gelöst. Die emulgatorhaltige Wasserphase wird dann unter Rühren in die emulgatorhaltige Ölphase eingetragen und emulgiert. Im Unterschied dazu wird im erfindungsgemäßen Verfahren die Ölphase in ein wässriges Emulgatorkonzentrat gegeben. Unter laminaren Strömungsbedingungen bildet sich ein Gel aus. Gele weisen erhebliche strukturelle Unterschiede zu Emulsionen auf, welche unterschiedliche physikalische Eigenschaften bewirken. Die DE 10346515 A1 offenbart lediglich ein übliches Emulsionsverfahren, bei denen die Ölphase ohne weitere Zwischenstufe in der Wasserphase emulgiert wird.

Adler-Nissen et al.: "Apparatus for Emulsion Production in Small Scale and Under Controlled Shear Conditions"; Trans IChemE, Part C, Food and Bio-products Processing; 2004, 82 (C4); pp. 311-319 offenbaren ein Verfahren zur Herstellung von Mayonnaise unter Verwendung von Eigelb als Emulgator, Wasser und Rapsöl. Hinweise auf das erfindungsgemäße Verfahren mit der Zwischenstufe eines Gelkonzentrates werden nicht gegeben.

Die DE 10 2004 031 550 A1 offenbart ein Verfahren zur Herstellung von Emulsionen, in dem ein Emulsionskonzentrat mit Wasser und weiteren Ölkomponenten verdünnt wird. Es werden keinerlei Angaben zur Herstellung des Emulsionskonzentrates gemacht.

Die EP 1 524 029 A1 offenbart ein Verfahren zur Herstellung von O/W-Emulsionen, in denen eine Emulgator/Ölphasenmischung in weiteren Ölphasenkomponenten gelöst und anschließend in der Wasserphase emulgiert wird. Zur Herstellung der Emulsionen werden die üblichen Heiß- oder Kaltprozesse eingesetzt.

Um volle Flexibilität sowohl hinsichtlich der Emulgatoren als auch hinsichtlich der Ölphase zu erhalten, ist es Aufgabe der vorliegenden Erfindung ein selbstemulgierendes Konzentrat zu schaffen, das es erlaubt, die Emulgatoren bzw. Tenside frei zu wählen, wobei eine breite Auswahl der zu verwendenden Ölkomponenten und natürlich auch der Elektrolyte und ihrer Konzentrationen möglich wird. Eine breite Auswahl einsetzbarer Hydrokolloide wäre hinsichtlich der erwünschten Viskosität wie auch des Hautgefühls und der ggf. gewünschten Wasserfestigkeit der getrockneten Emulsionen ebenfalls wünschenswert.

Gleichzeitig wäre es wünschenswert, ein Konzentrat zu erhalten, das es erlaubt, aufwendige Homogenisierungsvorrichtungen, wie z.B. Rotor-Stator- oder Hochdruckhomogenisatoren, zu vermeiden, und die Verarbeitung bei höheren Temperaturen dazu aber insbesondere auch bei Raumtemperatur erlaubt. Nach einer Ausgestaltung sollte eine möglichst geringe mittlere Tropfengröße - Mindestanforderung mittlere Tropfendurchmesser unter 1 Mikrometer - zu erzielen sein, ohne auf die Zwischenstufe eines selbstemulgierenden Konzentrats verzichten zu müssen.

Weiterhin sollen Konzentrate zur Verfügung gestellt werden, aus denen sich durch Wasserverdünnung und Zugabe weiterer Stoffe Emulsionen gleich bleibender Qualität herstellen lassen wobei die Herstellung sowohl kontinuierlich als auch chargenweise erfolgen kann. Für beide Herstellverfahren soll eine schnelle, sichere Qualitätskontrolle ermöglicht werden, um eine durchgehend hohe und gut vorhersagbare Emulsionsqualität zu gewährleisten.

Überraschend löst die vorliegende Erfindung das oben geschilderte Problem durch ein Verfahren zur Bereitstellung von selbstemulgierenden Gelkonzentraten und daraus erhältlichen Nano- oder Makroemulsionen. Das Verfahren ist durch folgende Schritte gekennzeichnet:
(a) Bereitstellen einer Emulgatorkonzentrates (A) enthaltend zumindest
   (A.1) zu 0,1 bis 75 Gew.%, ein oder mehrere Polyole (P),
   (A.2) zu 5 bis 80 Gew.% Wasser (W) und
   (A.3) zu 5 und 40 Gew.% und insbesondere zwischen 10 bis 30 Gew.%. ein oder mehrere ionische Tenside (I) und / oder ein oder mehrere nichtionische Tenside (N), vorzugsweise beide
      jeweils bezogen auf das Emulgatorkonzentrat (A);
(b) in Kontaktbringen einer Ölphase (O) mit dem Emulgatorkonzentrat (A) im laminaren Strömungsfeld, um ein selbstemulgierendes O/W Gelkonzentrat (G) mit einem Ölgehalt 60 bis 99 % und besonders bevorzugt 80 bis 98 Gew.% zu erhalten;
(c) Zusammenbringen des O/W Gelkonzentrats (G) mit Wasser, das auch weitere Zuschlagsstoffe enthalten kann, um sowohl selbsttätig, ohne Einwirken von Scherkräften, oder auch beschleunigt, unter Zuhilfenahme eines Rührers, aber im laminaren Strömungsfeld, eine O/W Makro- (M) oder Nanoemulsion (C) zu erhalten,wobei die Öl-in-Wasser-Dispersion eine Emulsion ist und die Ölphase (O) die disperse Phase ist.

Bevorzugt sind Nanoemulsionen, welche mittlere Tropfengrößen (bestimmt über statische Laserlichtstreuung gemäß DIN/ISO 13320) von unter 1.000 nm und bevorzugt kleiner 500 nm aufweisen.

Das Emulgatorkonzentrat (A) ist bei Raumtemperatur homogen aber nicht zwingend isotrop. Es darf über einen Zeitraum von 2 Stunden keine Separationserscheinungen zeigen und enthält 0,01 bis 99 Gew%, bevorzugt 1 bis 80 Gew%, insbesondere 5 bis 40 Gew% und ganz besonders bevorzugt 10 bis 30 Gew% bzw. 20 bis 30 Gew% Wasser. Der Anteil des Emulgatorkonzentrats (A) am selbstemulgierenden Gelkonzentrat (G) beträgt insbesondere 1 bis 40 und ganz besonders bevorzugt 2 bis 20 Gew.%.

Der Tensidanteil der aus der spontanen Emulsion des selbstemulgierenden Gels (G) in Wasser resultierenden Emulsion liegt bei < 10 %, bevorzugt bei < 5 %, besonders bevorzugt bei < 3 % und ganz besonders bevorzugt brei < 2 %, Der Gehalt der Ölkomponente des selbstemulgierenden Gelkonzentrats (G) liegt bei 60 bis 99 Gew%, bevorzugt bei 80 bis 98 Gew% und besonders bevorzugt bei 84 bis 96 Gew%.

Die selbstemulgierenden Gelkonzentrate (G) können eine außerordentliche Lagerstabilität aufweisen und können analog der Masterbatches verwendet werden. Sie sind über einen breiten Temperaturbereich lagerstabil. Sie können sowohl klar als auch trüb sein.

Die erfindungsgemäß hergestellten selbstemulgierenden Gelkonzentrate (G) zeichnen sich dadurch aus, dass sie durch Zusammenbringen von ölkomponenten zum Emulgatorkonzentrat (A) herstellbar sind. Der Gehalt an Ölkompenenten liegt vorzugsweise über dem kritischen Phasenvolumenverhältnis, bei dem eine Inversion der Emulsion zu erwarten wäre, entsprechend der Definition von Ostwald (Wa. Ostwald, Beiträge zur Kenntnis der Emulsionen, Z. Kolloid, 6 (1910), 103-109).

Die Gelkonzentrate können nur durch Vermischung des Emulgatorkonzentrats (A) mit den Ölkomponenten unter Einhalten eines laminaren Strömungsbereiches hergestellt werden. Dabei kann sowohl chargenweise wie auch kontinuierlich gemischt werden, lediglich die Mischung im laminaren Strömungsbereich muss sichergestellt sein. Die Größe der Mischapparatur spielt ebenso wenig eine Rolle, es kann konventionelle Mischtechnik eingesetzt werden, die erfindungsgemäßen Emulsionen sind auch gut geeignet für die kontinuierliche Herstellung in Mikroprozeßapparaturen.

Trotz der recht hohen Ölkonzentration weisen die selbstemulgierenden Gelkonzentrate (G) eine erstaunlich hohe Leitfähigkeit auf: Laut H. Junginger et al., zeigen typische O/W Emulsionen unterhalb eines Wassergehaltes von 20 Gew.% keine Leitfähigkeit im mikro Siemens Bereich mehr [Aufbau und Entwicklung von Salben, Cremes und Emulsionen, Dermatikkurs II, Arbeitsgemeinschaft für Pharmazeutische Verfahrenstechnik (APV) e.V., H. Junginger, Mainz, 1983]. Anders die selbstemulgierenden Gelkonzentrate (G), die eine im mikro Siemens Bereich (größer 1 mikro Siemens) messbare Leitfähigkeit (bei 25 °C) aufweisen von z.B. der Größenordnung 3 mikro Siemens.

Die Herstellung der selbstemulgierenden Gele ist nur in dem Sinne temperaturabhängig, dass sich die Ölkomponenten homogen mischen lassen müssen. Bei der Herstellung des Gelkonzentrates (G) muss die Ölphase flüssig sein, das bestimmt die Herstelltemperatur. Abhängig von den eingesetzten Ölkomponenten ist eine erhöhte Temperatur notwendig.

Allgemein kann man feststellen, dass die erzielbare mittlere Tröpfchengröße der Nanoemulsion (C) durch Selbstemulgierung des Gelkonzentrates (G) in Wasser sich proportional zur Temperatur verhält. Die Viskosität der Nanoemulsion (C) lässt sich sowohl durch den Ölgehalt, Verdünnungsgrad, als auch durch Hydrokolloide bzw. Verdicker steuern. Dünnflüssige und sprühbare (sowohl als Pumpspray wie auch als Aerosolspray einsetzbar) Emulsionen lassen sich mit einer Ölphase (O) bis max. 50 Gew. % bezogen auf die Nanoemulsion (C), bevorzugt max. 40 Gew. % bezogen auf die Nanoemulsion (C) und besonders bevorzugt max. 30 Gew. % bezogen auf die Nanoemulsion (C) herstellen.

Als typische Kennwerte zur Charakterisierung der Nanoemulsion (C) sind das Verhältnis Ölphase (O) zu Tensid bzw. Emulgator (I+N = E) als Kennzahl Q definiert: O/E = Q. Erfindungsgemäß liegt Q zwischen 1 und 100, bevorzugt 5 bis 50 und besonders bevorzugt 7 bis 35.

Darüber hinaus ist die aus der Laserstreulicht basierten Bestimmung der Partikelgröße unter Annahme einer sphärischen Partikelstruktur ermittelte Oberfläche der Partikel (A_{P}) wie folgt definiert: A_{P} (emul.) = k * exp (k' + E), mit k' als Stoffkonstante, in m² Oberfläche der Partikel pro Gramm Emulsion. Für die erfindungsgemäßen Nanoemulsionen liegt i.d.R. Aₚ zwischen 5 und 2000, bevorzugt 10 bis 1000 und besonders bevorzugt 20 bis 800 m²/g.

In den erfindungsgemäßen Nanoemulsionen (C) weist die dispergierte Ölphase i.d.R. die Form kleiner Bruchstücke (Kompartimente), mit einem Median zwischen ca. 10 nm und 10 Mikrometern, bevorzugt zwischen 200 nm und 5 Mikrometer, besonders bevorzugt zwischen 300 nm und 1,5 Mikrometer und ganz besonders bevorzugt von unter einem Mikrometer auf.

Sie sind stabil in Tau-Gefrier-Zyklen (5 mal -18 bis 40 °C mit mindestens 12 h bei jeder Temperatur) und lagerstabil bei Raumtemperatur sowie bei höheren Temperaturen wie 40 und 50 °C.

Die besondere Struktur der dispergierten Ölphasenpartikel der erfindungsgemäßen Nanoemulsionen hat einen von konventionellen Emulsionen unterschiedlichen Verlauf der Trocknung zur Folge. Nimmt man die Leitfähigkeit während der Trocknung auf, so unterscheidet sich ihr Verlauf deutlich von dem konventioneller Emulsionen, und zwar insoweit, dass ein Gleichgewichtswert der Leitfähigkeit (der nicht gleich Null ist) sich schon bei deutlich kürzeren Trocknungsdauern einstellt.

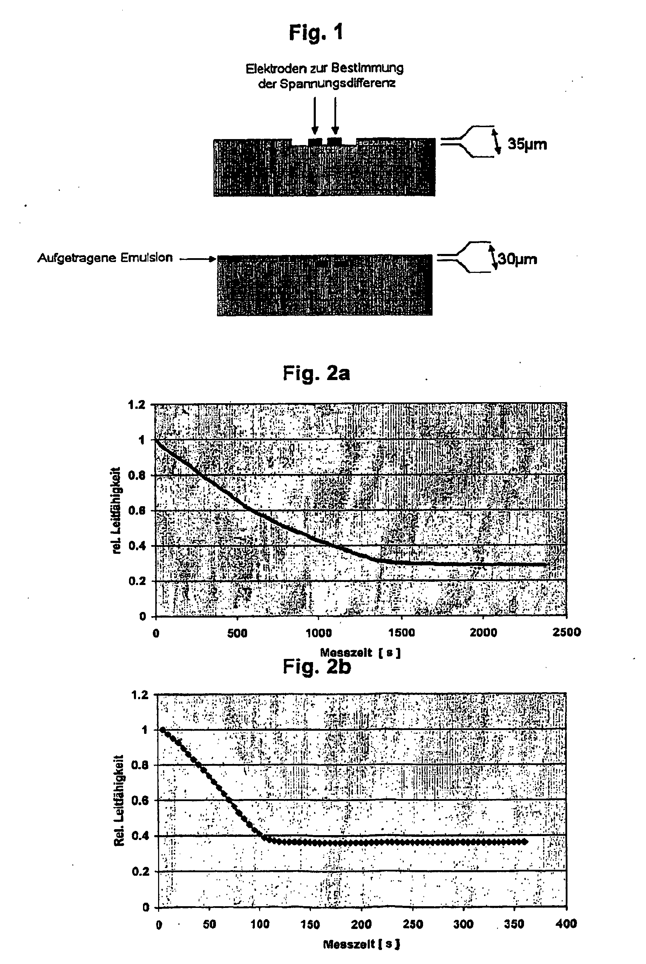

Die entsprechende Messeinrichtung ist in Fig. 1 dargestellt,
Fig. 2 zeigt Leitfähigkeitskurven von Trocknungsverläufen konventioneller O/W Emulsion (Fig. 2a) und erfindungsgemäßer Nanoemulsionen (Fig. 2b).
In Fig. 3 und 4 sind Gefrierbrüche dargestellt.

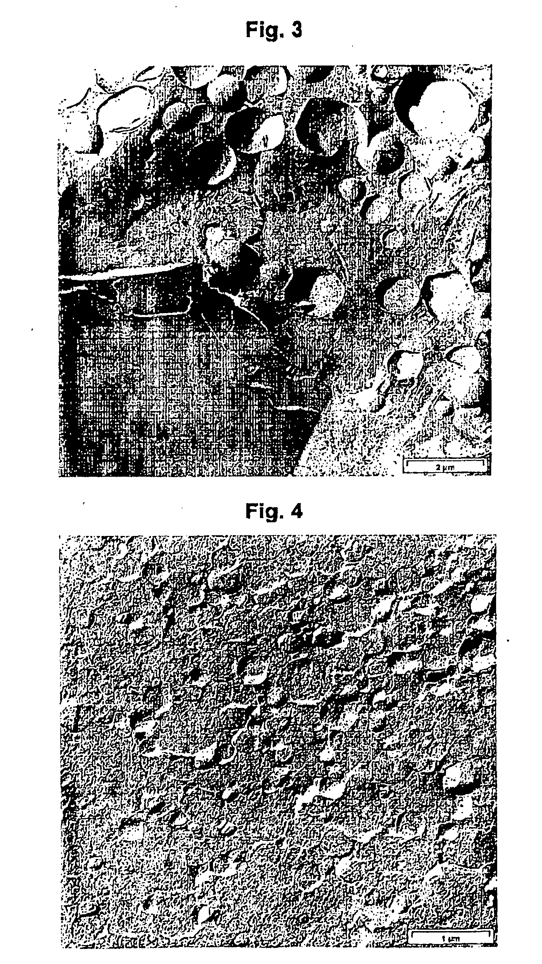

Fig. 3. zeigt den Gefrierbruch (TEM nach Gefrierbruch) einer konventionellen O/W Emulsion, die wie folgt zusammengesetzt ist:

| | | |
|---|---|---|
| A) | Imwitor 380 (Glyceryl Cocoate/Citrate/Lactate) | 3.0% |
| | Miglyol 812 (Caprylic/Capric Triglyceride) | 5.0% |
| | Cosmacol EMI (Di-C12-13 Alkyl Malate) | 2.5% |
| | Cosmacol EOI (C-12-13 Alkyl Octanoate) | 3.0% |
| | Avocado Öl (Persea Gratissima Oil) | 3.0% |
| | Cyclomethicone | 1.5% |
| B) | Water (Aqua) demin. | ad 100.0 % |
| | Xanthan Gum | 0.5 % |
| | Glycerin | 6.0% |
| C) | Tocopheryl Acetate | 1.0% |
| | Fragrance | q.s. |
| | Preservative | q.s. |

und Fig. 4 zeigt den Gefrierbruch des erfindungsgemäßen Beispiels 4.

Die Phase A) und B) werden getrennt auf 70 °C erhitzt und Phase B) unter Homogenisierung (mit Rotor-Stator-Mischer, Ultra Turrax) für ca. 2 Minuten gemischt. Anschließend wird abgekühlt und bei 30 °C Phase C) zugegeben und noch einmal für 1 Minute wie oben beschrieben homogenisiert. Die mittlere Tropfengröße liegt bei 1. 5 Mikrometern.

Bedingt durch die besondere Struktur der dispergierten Ölpartikel ist i.d.R. ein beim Eintrocknen der Nanoemulsionen (C) überraschend auftretender Memoryeffekt zu beobachten: Lässt man eine Nanoemulsion (C) bei 25 °C und Atmosphärendruck eintrocknen, so verdunstet das Wasser so lange bis der Zustand des ursprünglichen Gelkonzentrates (G) erreicht ist. Dieses bleibt unter den genannten Bedingungen wenigstens 24 Stunden stabil und ist, wie direkt nach der Herstellung, selbstemulgierend. Dabei stellt sich auch wieder die ursprüngliche - für das entsprechende Gelkonzentrat charakteristische - Partikelgrößenverteilung der dispergierten Ölphase ein. Dieser Vorgang lässt sich beliebig oft ohne Änderung der Partikelgrößenverteilung wiederholen. Die erfindungsgemäßen Nanoemulsionen (C) unterscheiden sich hinsichtlich dieses Memoryeffektes signifikant von konventionellen Emulsionen, die sich bei solch einem Eintrocknungsvorgang irreversibel trennen.

Die von Emulsionen nach dem Stand der Technik abweichende innere Struktur der dispergierten Ölphasenpartikel der erfindungsgemäßen Nanoemulsionen (C) zeichnen sich durch den Aufbau eines zusammenhängenden Films bei der Trocknung aus. Diese Eigenschaft erweist sich sowohl für Sonnenschutzformulierungen wie auch für Lacke und Farben vorteilhaft, weil hierdurch Glanz und Kratzfestigkeit deutlich erhöht werden können.

Bei dem ionischen Tensid I, kann es sich sowohl um anionische, kationische als auch um amphotere Tenside handeln, die entweder einzeln oder in Kombinationen eingesetzt werden können. Sich gegenseitig neutralisierende und dann ausfallende Kombinationen von Tensiden sind nicht geeignet.

Anionische Tenside in (A), die spontan bei Raumtemperatur dazu neigen, in Konzentrationsbereichen <20 % lamellare Phasen in Wasser auszubilden, wie zum Beispiel Natrium Lauroyllactylat (INCI Nomenklatur), Natrium Cetylsulfat, Natrium Stearoyllactylat (INCI Nomenklatur), werden insbesondere in Kombination mit anderen Tensiden verwendet und sind für den alleinigen Einsatz nicht geeignet

Der Gehalt an Polyol im Emulgatorkonzentrat (A) liegt zwischen 0 und 80 Gew.%, bevorzugt zwischen 0,1 und 75 Gew.% besonders bevorzugt bei 10 bis 60 Gew.% und ganz besonders bevorzugt bei 20 bis 50 Gew.%, wobei bei Verwendung nur eines Tensidtyps (I oder N) die geeignete Konzentration vorzugsweise größer 30 Gew.% beträgt.

Ohne Verwendung von Polyolen ist die Stabilität der Nanoemulsionen jedoch im Tau-Gefrier-Zyklus oft unbefriedigend. Auch der Einsatz höherer Elektrolytkonzentrationen bedingt die Anwesenheit von Polyolen. Die Polyole weisen vorzugsweise 2 bis 1000 Kohlenstoffatome im (ggf. verzweigten) Kohlenwasserstoffrest und vorzugsweise 2 bis 50, bevorzugt 2 bis 20, besonders bevorzugt 2 bis 10 und ganz besonders bevorzugt 3 bis 6, Hydroxylgruppen auf. Geeignete Polyole sind z.B. Alkylenglykole wie Ethylen-, Propylen-, Butylen-, Pentylen- sowie Hexylenglykol sowie ihre jeweiligen Isomeren (z.B. Neopentylglykol), wie auch Triole, wie Glycerin, und höhere, wie Trimethylolpropan, Pentaerythrit, Polyglycerine, Glucoside und Polyglucoside, Saccharide und ihre jeweiligen Alkylderivate und deren Mischungen. Geeignet sind auch Polyvinylalkohole und Polyfructose.

Glycerin hat sich als das vielseitigste Polyol gezeigt, doch sind eine ganze Reihe anderer Verbindungen mit mehren Hydroxygruppen auch geeignet, wobei dem Glycerin ähnliche Verbindungen wie polare Glykole besonders geeignet sind. Ethylenglykol, Propylenglykol, Butylenglykol oder Pentylenglykol oder Derivate des Glycerins aber auch PEG Derivate sind besonders geeignet.

Lactose, Dextrose, Propylenoxid Block-Copolymere ebenso wie aminofunktionalisierte Propylenoxid Derivate sind recht gut geeignet, während Sorbitol nur mit einer gewissen Einschränkung der Emulgatoreffizienz verwendbar ist, d.h. bei der Verwendung von Sorbitol ist Q < 15 zu erreichen.

Die Herstellung des Emulgatorkonzentrates A erfolgt durch die Mischung der Komponenten, wobei es keine Einschränkungen hinsichtlich der Mischwerkzeuge und Temperaturbereiche gibt

Als Ölphase bzw. die Ölphase bildende Ölkomponenten im Sinne der Erfindung werden Stoffe mit einer Grenzflächenspannung zu demineralisiertem Wasser bei 25 °C von größer als 3 mN*m⁻¹, bevorzugt 5 bis 69 mN*m⁻¹, verstanden.

Besonders geeignete Ölkomponenten, auf die die obengenannte Definition zutrifft, sind Paraffine, Ester, auch Mischester zwischen Siliconen bzw. funktionalisierten Siliconen und organischen Komponenten jedoch insbesondere Glyceride und ihre Derivate. Dimethicone mit Viskositäten > 0,0001 mPas und auch hochviskose Siliconöle, mit Viskositäten > 60 Pas (jeweils bei 25°C) können ohne besondere Werkzeuge, die hohe Scherung ermöglichen, zu selbstemulgierenden Gelen verarbeitet werden. Daneben genannt sind auch Destillierrückstände der Petrochemie, Gatschen und Bitumen.

Der Begriff "selbstemulgierend" beschreibt einen spontanen Emulsionsprozess, der bei Kontakt des selbstemulgierenden O/W Gels mit Wasser abläuft. D.h. bringt man das Gel (G) mit zusätzlichem Wasser in Kontakt, emulgiert sich die Ölphase, bisweilen nach einer Initialisierungsperiode von maximal ein paar Minuten in Form kleiner Bruchstücke, mit einem Median zwischen ca. 100 nm und 10 Mikrometern, bevorzugt zwischen 200 nm und 5 Mikrometer, besonders bevorzugt zwischen 300 nm und 1,5 Mikrometer und ganz besonders bevorzugt von unter einem Mikrometer ohne zusätzliches Rühren oder andere mechanische Unterstützung innerhalb von wenigen Minuten bis mehreren Stunden selbst. Die spontane Emulsion führt zu stabilen O/W Emulsionen.

Der Gehalt an Ölphase in der Nanoemulsion (C) liegt zwischen 0.1 und 70 Gew% bevorzugt 5 bis 60 % und besonders bevorzugt 10 bis 50 Gew%.

Bei Tensidmischungen (I) plus (N) sind Gewichts-Verhältnisse von (I) zu (N) zwischen 0,01 zu 3 bis 3 zu 0,01 besonders geeignet. Es muss jedoch nur jeweils ein Tensid (I) oder (N) vorhanden sein, bevorzugt ist es jedoch eine Kombination von (I) und (N) im Emulgatorkonzentrat (A) einzusetzen. Bei Verwendung unterschiedlicher Tensidtypen werden stabilere Nanoemulsionen und auch geringere erzielbare Tropfengrößen erhalten.

Nichtionische Tenside oder Tensidkombinationen mit einen HLB (berechnet nach Griffin, J. Soc. Cosmet. Chem. 1, (1949) 311-326) größer gleich 10 sind insbesondere geeignet, sei es allein oder weiter bevorzugt in Kombination mit einem ionischen Tensid. Weiterhin sind nichtionische Tenside mit besonders hoher Polarität geeignet, auch für den alleinigen Einsatz in (A), beispielsweise C8 bis C14Diamidethoxylate, wie solche basierend auf C12/C14 oder C8/C10 Diamiden, mit mindestens 30 Ethylenglykoleinheiten.

Nachfolgend seien Beispiele für geeignete Tenside aufgeführt:
Geeignete nichtionische Tenside umfassen:
   Tenside, die mit einem Alkoholrest terminieren, wie z. B.:
      (1.a) C₁- bis C₄- Alkoxylate, einschließlich deren Mischungen, von verzweigten oder linearen, gesättigten oder ein- bis dreifach ungesättigten C10- bis C22- Alkoholen, insbesondere C12- bis C18- Fettalkoholethoxylate, ethoxylierte Wollwachsalkohole, Polyethylenglycolether der allgemeinen Formel R-O-(-CH₂-CH₂-O-)ₙ-R', z. B. Fettalkoholethoxylate aus der Gruppe polyethoxylierter bzw. polypropoxylierter bzw. polyethoxylierter und polypropoxylierter Produkte, umfassend, Monoalkohole wie ethoxylierte Stearylalkohole, Cetylalkohole, Cetylstearylalkohole und andere Polyglykole wie Ethylenoxid-Propylenoxid Block-Co-Polymere, und Polyvinylalkohole sowie ethoxylierte Sorbitanester, Cholesterinethoxylate oder Alkylpolyglycoside mit Polymerisationsgraden größer 1 und N-Alkylpyrrolidonderivate.
      (1.b) Fettalkoholpropoxylate der allgemeinen Formel R-O-(-CH₂-CH(CH₃)-O-)ₙ-H, Polypropylenglycolether der allgemeinen Formel R-O-(-CH₂-CH(CH₃)-O-)ₙ-R', der propoxylierten Wollwachsalkohole, veretherte Fettsäurepropoxylate R-COO-(-CH₂-CH(CH₃)-O-)ₙ-R', veresterte Fettsäurepropoxylate der allgemeinen Formel R-COO-(-CH₂-CH(CH₃)-O-)ₙ-C(O)-R', Fettsäurepropoxylale der allgemeinen Formel R-COO-(-CH₂-CH(CH₃)-O-)ₙ-H, Polypropylenglycolglycerinfettsäureester, propoxylierte Sorbitanester, Cholesterinpropoxylate, propoxylierte Triglyceride der Alkylethercarbonsäuren der allgemeinen Formel R-O-(-CH₂-CH(CH₃)O-)ₙ-CH₂-COOH, Fettalkoholethoxylate(X)/propoxylate(Y) der allgemeinen Formel R-O-Xₙ-Yₘ-H, Polypropylen(Y)/ethylen(X)glycolether der allgemeinen Formel R-O-XₙYₘ-R', veretherte Fettsäurepropoxylate(Y)/ethoxylate(X) der allgemeinen Formel R-COO-XₙYₘ-R' und/oder Fettsäureethoxylate(X)/propoxylate(Y) der allgemeinen Formel R-COO-XₙYₘ-H.
      (1.c) Monoglycerinester gesättigter und/oder ungesättigter, verzweigter und/oder unverzweigter Alkancarbonsäuren einer Kettenlänge von 8 bis 32, insbesondere 12 bis 18 C-Atomen, Diglycerinester gesättigter und/oder ungesättigter, verzweigter und/oder unverzweigter Alkancarbonsäuren einer Kettenlänge von 8 bis 32, insbesondere 12 bis 18 C-Atomen, Tri- bis Decaglycerinester gesättigter und/oder ungesättigter, verzweigter und/oder unverzweigter Alkancarbonsäuren einer Kettenlänge von 8 bis 32, insbesondere 12 bis 18 C-Atomen, Monoglycerinether gesättigter und/oder ungesättigter, verzweigter und/oder unverzweigter Alkohole einer Kettenlänge von 8 bis 32, insbesondere 12 bis 18 C-Atomen, Di-, tri- bis deca-glycerinether gesättigter und/oder ungesättigter, verzweigter und/oder unverzweigter Alkohole einer Kettenlänge von 8 bis 32, insbesondere 12 bis 18 C-Atomen, Propylenglycolester gesättigter und/oder ungesättigter, verzweigter und/oder unverzweigter Alkancarbonsäuren einer Kettenlänge von 8 bis 32, insbesondere 12 bis 18 C-Atomen sowie Sorbitanester gesättigter und/oder ungesättigter, verzweigter und/oder unverzweigter Alkancarbonsäuren einer Kettenlänge von 8 bis 32, insbesondere 12 bis 18 C-Atomen.
         Konkrete Beispiele für diese Gruppe sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol-2-stearylether (Steareth-2), Glycerylmonolaurat, Glycerylmonocaprinat, Glycerylmonocaprylat, Alkylphenolpolyglycolether (z. B. Triton X), Glycerylmono- und diester der Guerbetcarbonsäuren von C12 bis C 32, bevorzugt C12 bis C 24, Zuckerderivate (Ester und/oder Ether von Glucose, Saccharose und anderen Zuckern), Kondensationsprodukte von aliphatischen Alkoholen mit 8 bis 18 Kohlenstoffatomen, entweder in geradkettiger oder verzweigtkettiger Konfiguration, mit Ethylenoxid, z. B. ein Kokosnussalkohol-Ethylenoxid-Kondensat mit 10 bis 30 Mol Ethylenoxid pro Mol Kokosnussalkohol, wobei die Kokosnussalkoholfraktion 10 bis 14 Kohlenstoffatome aufweist.
      (1.d) Alkylpolysaccharid(APS)-Tenside (zum Beispiel Alkylpolyglycoside), gegebenenfalls kann eine Polyalkylenoxid-Gruppe, die die hydrophoben und hydrophilen Reste verknüpft, vorliegen; und die C8- bis C32-Alkylgruppe, bevorzugt C8 bis C18, (d. h. der hydrophobe Rest) kann gesättigt oder ungesättigt, verzweigt oder unverzweigt und unsubstituiert oder (beispielsweise mit Hydroxy oder cyclischen Ringen) substituiert sein.
      (1.e) Polyethylenglykol(PEG)-Glyceryl-Fettester, wie jene der Formel R(O)OCH₂CH(OH)CH₂(OCH₂CH₂)nOH, worin n im Durchschnitt 5 bis 200, vorzugsweise etwa 20 bis etwa 100 ist, und R ein aliphatisches Hydrocarbonyl mit etwa 8 bis etwa 20 Kohlenstoffatomen ist, wie z. B. Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat und/oder Polyethylenglycol(18)glyceryloleat/cocoat,
      (1.f) ethoxylierte Cholesterinderivate wie Polyethylenglycol(30)cholesterylether, oder auch Polyethylenglycol(25)sojasterol.
      (1.g) ethoxylierte Triglyceride wie Polyethylenglycol-Nachtkerzen-Glyceride und Polyethylenglycol-Kokos-, Soja-, Babassu- und Mandelölglyceride.
      (1.h) Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat und / oder Polyethylenglycol(20)sorbitanmonooleat.
(2) Tenside, die mit Carbonsäuren terminieren, wie z. B.:
   (2.a) C2- bis C4- Alkoxylate, einschließlich deren Mischungen, der Mono- und Difettsäureglyceride, der Fettsäureethoxylate der allgemeinen Formel R-COO-(-CH₂-CH₂-O-)n-H, veretherte Fettsäureethoxylate der allgemeinen Formel R-COO-(-CH₂-CH₂-O-)n-R', veresterte Fettsäureethoxylate der allgemeinen Formel R-COO-(-CH₂-CH₂-O-)n-C(O)-R', Partialfettsäureester und Fettsäureester mehrwertiger Alkohole und deren ethoxylierte Derivate wie Polyethylenglycolglycerinfettsäureester, Polyethylenglycolstearylether mit 12 bis 20 Polyethylenglycol-Einheiten, Polyethylenglycolisostearylether mit 12 bis 20 Polyethylenglycol-Einheiten, der Polyethylenglycololeate mit 12 bis 20 Polyethylenglycol-Einheiten, Glycerylmonostearate, Sorbitanstearate, Glycerylstearylcitrate, Sucrosestearate, ethoxylierte Triglyceride, Polyoxyethylensorbitolfettsäureester, Fettsäureamide, Fettsäurealkanolamide, veretherte Fettsäurepropoxylate der allgemeinen Formel R-COO-(-CH₂-CH(CH₃)-O-)ₙ-R', veresterte Fettsäurepropoxylate der allgemeinen Formel R-COO-(-CH₂-CH(CH₃)-O-)ₙ-C(O)-R' und
   (2.b) Alkylethercarbonsäuren der allgemeinen Formeln R-O-(-CH₂-CH₂-O-)ₙ-CH₂-COOH und R-O-(-CH₂-CH₂-(CH₃)-O-)ₙ-CH₂-COOH, worin n bzw. der Alkoxylierungsgrad jeweils eine Zahl von 5 bis 30, insbesondere 8 bis 18, ist. Ebenso geeignet sind gemischt alkoxylierte Ethercarbonsäure der allgemeinen Formeln R-O-(-CH2-CH₂-O-)n-(-CH₂-CH₂-(CH₃)-O-)m-CH₂-COOH, worin n und m bzw. der Alkoxylierungsgrad jeweils eine Zahl von 5 bis 30, insbesondere 8 bis 18, sind. R ist linear oder verzweigt, gesättigt bis dreifach ungesättigt mit C8 bis C 32, bevorzugt C 8 bis C18. Zur Neutralisation werden bevorzugt Alkali- und Erdalkali sowie Alkanolamine eingesetzt.
(3) Tenside mit anderen Eigenschaften, wie z. B.:
   (3.a) Polyethylenoxid-Kondensate von Alkylphenolen, die z. B. Kondensationsprodukte sind von Alkylphenolen mit einer Alkylgruppe von 6 bis 20 Kohlenstoffatomen in entweder einer linearen oder verzweigten Konfiguration, mit Ethylenoxid, wobei das Ethylenoxid in Mengen von gleich etwa 10 bis etwa 60 Mol Ethylenoxid pro Mol Al-kylphenol vorhanden ist,
   (3.b) Kondensationsprodukte (Blockstruktur oder statistisch verteilt) von Ethylenoxid mit dem Produkt aus der Reaktion von Propylenoxid und Ethylendiaminen; N,N'-Diacylalkylendiaminalkoxylate, ethoxylierte Fettamine und alkoxylierte N-Acylamide wie auch N-Acyl-N-Alkylamidalkoxylate sind ebenfalls geeignet,
   (3.c) langkettige tertiäre Aminoxide der Formel [RR'R"N-O], worin R einen Alkyl-, Alkenyl- oder Monohydroxyalkylrest von 8 bis 18 Kohlenstoffatomen, von 0 bis 10 Ethylenoxideinheiten und von 0 bis 1 Glyceryleinheit enthält, und R' und R" 1 bis 3 Kohlenstoffatome und 0 bis 1 Hydroxygruppen enthalten, z. B. Methyl-, Ethyl-, Propyl-, Hydroxyethyl- und/oder Hydroxypropylreste,
   (3.d) langkettige tertiäre Phosphinoxide der Formel [RR'R"P-O], worin R einen Alkyl-, Alkenyl-oder Monohydroxyalkylrest im Bereich von etwa 8 bis etwa 18 Kohlenstoffatomen Kettenlänge, 0 bis 10 Ethylenoxideinheiten und von 0 bis 1 Glyceryleinheit enthält, und R' und R" jeweils Alkyl- oder Monohydroxyalkylgruppen mit 1 bis 3 Kohlenstoffatomen sind und
   (3.e) langkettige Dialkylsulfoxide, enthaltend einen kurzkettigen Alkyl- oder Hydroxyalkylrest von 1 bis 3 Kohlenstoffatomen (üblicherweise Methyl) und eine lange hydrophobe Kette, welche Alkyl-, Alkenyl-, Hydroxyalkyl- oder Ketoalkylreste mit 8 bis 20 Kohlenstoffatomen, 0 bis 10 Ethylenoxideinheiten und 0 bis 1 Glyceryleinheit enthält,
   (3.f) nichtionische Geminitenside, auch Dimer- oder Zwillingstenside genannt, die dadurch gekennzeichnet sind, dass zwei Tensideinheiten bestehend aus einer hydrophoben Gruppe und einer hydrophilen Gruppe durch einen Spacer nahe der hydrophilen Gruppe miteinander verbunden sind. Beispielsweise sind N,N'-Dialkyl-N,N'-dialkoxylate besonders geeignet.
   (3.g) Sorbitanfettsäureester, Polyoxyethylensorbitanfettsäureester, Lactobionsäureamide, Gluconamide, N-Methylgluconamide mit einem Alkylrest von C6 bis C32, bevorzugt C8 bis C 18, linear oder verzweigt, gesättigt oder ungesättigt;
   (3.h) in Kombination mit einem sehr polaren anionischen Tensid, wie Alkylethersulften oder Alkylsulfaten oder kurzkettigen Sulfosuccinaten, lassen sich auch kurzkettige, bevorzugt verzweigte Fettalkohole mit C6- bis C15-, besonders bevorzugt C8- bis C13-Resten einsetzen. Ganz besonders geeignet sind Alkohole, die unter dem Markennamen Safol 23, Marlipal 013, Isalchem 123 und Isalchem 125, sowie Marlipal 031 angeboten werden. Besonders geeignet sind (INCI Namen) Sodium Laureth-Sulfate, MIPA- und TIPA-Laureth Sulfate jeweils mit 2 Ethylenglycoleinheiten wie auch die mit 3 Ethylenglykoleinheiten versehenen Analoga.

Eine weitere Besonderheit in Kombination mit sehr polaren anionischen Tensiden (wie oben beschrieben) bilden Alkanollactate von bevorzugt monoverzweigten Oxo-Alkoholen wie das C12- bis C13- Alkyllactat (INCI Name) Cosmacol ELI. Geeignet ist ebenfalls das C12-C15 Analogon.

### Anionische Tenside

(1) Fettsäuren mit 8 bis 30 Kohlenstoffatomen, Glycerin- Mono- und Diester gesättigter und/oder ungesättigter, verzweigter und/oder unverzweigter Alkancarbonsäuren einer Kettenlänge von 8 bis 32, insbesondere 12 bis 18 C-Atomen, Diglycerinester gesättigter und/oder ungesättigter, verzweigter und/oder unverzweigter Alkancarbonsäuren einer Kettenlänge von 8 bis 32, insbesondere 12 bis 18 C-Atomen, Monoglycerinether gesättigter und/oder ungesättigter, verzweigter und/oder unverzweigter Alkohole einer Kettenlänge von 8 bis 32, insbesondere 12 bis 18 C-Atomen, Diglycerinether gesättigter und/oder ungesättigter, verzweigter und/oder unverzweigter Alkohole einer Kettenlänge von 8 bis 32, insbesondere 12 bis 18 C-Atomen, Propylenglycolester gesättigter und/oder ungesättigter, verzweigter und/oder unverzweigter Alkancarbonsäuren einer Kettenlänge von 8 bis 32, insbesondere 12 bis 18 C-Atomen sowie Sorbitanester gesättigter und/oder ungesättigter, verzweigter und/oder unverzweigter Alkancarbonsäuren einer Kettenlänge von 8 bis 32, insbesondere 12 bis 18 C-Atomen, die mit Milch- und Zitronen- oder Weinsäure verestert worden sind und darüber hinaus noch teilweise neutralisiert sein können (Imwitor 380, 375, 377, 372 P). Ebenso zählen dazu unvollständig veresterte Oligo- oder Polycarbonsäuren, dazu gehören auch Fruchtsäuren (Zitronensäure, Weinsäure, Äpfelund Apfelsäure) mit ihren Mono- oder Diestern von linearen oder verzweigten, gesättigten oder einfach oder mehrfach ungesättigten C6 bis C40 Alkoholen deren verbleibende Carbonsäuregruppe anschliessend neutralisiert worden sind. Zum Neutralisieren finden Natrium, Kalium, Monoethanolammonium und Monoisopropanolammonium Kationen bevorzugt Verwendung. Ebenso gehören Bernstein- und Adipinsäure, Maleinsäure, Fumarsäure dazu.
(2) Alkylethersulfate bzw. die diesen Sulfaten zugrundeliegenden Säuren der allgemeinen Formel R-O-(C₂H₄O)ₓ(-CH₂-CH(CH₃)-O-)ₙ-SO₃-H und Alkyl- und Alkylethersulfate mit den jeweiligen Formeln ROSO₃M und RO(C₂H₄O)ₓSO₃M, worin R Alkyl von etwa 8 bis etwa 32, vorzugsweise 12 bis 18 Kohlenstoffatomen ist, und linear oder einfach bis mehrfach verzweigt sein kann, x 1 bis 10 ist, und M ein Kation ist, wie Ammonium, Alkanolamine (z. B. Triethanolamin, Mono- und Triethanol und Mono- und Triisopropanolamin), einwertige Metallkationen (Natrium und Kalium) und mehrwertige Metallkationen, wie (Magnesium und Calcium). Die Alkylethersulfate werden typischerweise als Kondensationsprodukte von Ethylenoxid und einwertigen Alkoholen mit 8 bis 24 Kohlenstoffatomen hergestellt. Die Alkohole können aus Fetten, z. B. Kokosnussöl oder Talg, abgeleitet sein oder können synthetisch sein. Laurylalkohol und geradkettige Alkohole, welche aus Kokosnussöl abgeleitet sind, ebenso Oxoalkohole mit C12-C13, einfach verzweigt und C13 Alkylketten auf Basis von Buten-Trimerisation oder Propen-Tetramerisation, werden hierin bevorzugt.
   Derartige Alkohole werden mit zwischen 0 und 10 und insbesondere 3 molaren Anteilen Ethylenoxid umgesetzt, und die resultierende Mischung von molekularen Spezies mit beispielsweise einem Durchschnitt von 3 Mol Ethylenoxid pro Mol Alkohol wird sulfatiert und neutralisiert.
   Konkrete Beispiele von Alkylethersulfaten sind die Natrium- und Ammoniumsalze von Kokossnussalkyltriethylenglykolethersulfat, Talgalkyltriethylenglykolethersulfat und Talgalkylhexaoxyethylensulfat oder von Succinaten, wie z. B. Dinatrium-N-octadecylsulfosuccinnat, Dinatriumlaurylsulfosuccinnat, Diammoniumlaurylsulfosuccinnat, Tetranatrium-N-(1,2-dicarboxyethyl)-N-octadecylsulfosuccinnat, oder die Diamylester der Natriumsulfobernsteinsäure, Dihexylester der Natriumsulfobernsteinsäure und Dioctylester der Natriumsulfobemsteinsäure.
   Weiter bevorzugte Alkylethersulfate sind diejenigen, umfassend eine Mischung von individuellen Verbindungen, wobei die Mischung eine durchschnittliche Alkylkettenlänge von 10 bis 18, bevorzugt 12 bis 16 Kohlenstoffatomen und einen durchschnittlichen Ethoxylierungsgrad von 1 bis 10, bevorzugt 1 bis 4 Mol Ethylenoxid aufweist. Beispiele sind, Ammoniumlaurethsulfat, Triethylaminlaurethsulfat, Triethanolaminlaurethsulfat, Monoethanolaminlaurethsulfat, Diethanolaminlaurethsulfat, Laurinmonoglycerid-Natriumsulfat, Natriumlaurethsulfat, Kaliumlaurylsulfat, Kaliumlaurethsulfat, Natriumlaurylsarcosinat, Natriumlauroylsarcosinat, Laurylsarcosin, Cocoylsarcosin, Ammoniumcocoylsulfat, Ammoniumlauroylsulfat, Natriumcocoylsulfat, Natriumlauroylsulfat, Kaliumcocoylsulfat, Triethanolaminlaurylsulfat, Triisopropylaminlaurylsulfat, Monoethanolamincocoylsulfat, Monoethanolaminlaurylsulfat, Natriumtridecylbenzolsulfonat und Natriumdodecylbenzolsulfonat und Natriumlaurethsulfat, sowie Natrium-, Kalium- und Monoethanolamin, Monoisopropanolamin Salze der C12 bis C32 Guerbetsäuren (erzeugen keine flüssigkristallinen Phasen und können allein eingesetzt werden). Es ist bevorzugt Alkylsulfate nicht einzusetzen.
(3) Andere geeignete anionische Tenside sind die wasserlöslichen Salze von organischen Schwefelsäurereaktionsprodukten (Sulfonate) der allgemeinen Formel [R'-SO₃-M], worin R' gewählt wird aus der Gruppe, bestehend aus linearen oder verzweigten, gesättigten aliphatischen Kohlenwasserstoffresten mit 8 bis 24, vorzugsweise 10 bis 18 Kohlenstoffatomen und wobei M ein Kation ist Beispiele solcher Tenside sind die Salze eines organischen Schwefelsäurereaktionsproduktes eines Kohlenwasserstoffs der Methan-Reihe, einschließlich Iso-, Neo- und n-Paraffinen mit 8 bis 24 Kohlenstoffatomen, vorzugsweise 12 bis 18 Kohlenstoffatomen, und einem Sulfonierungsmittel, z. B. SO₃, H₂SO₄, Oleum erhalten gemäß bekannten Sulfonierungsverfahren, einschließlich Bleichen und Hydrolyse. Bevorzugt werden sulfonierte Alkalimetall- und Ammonium-C₁₀₋₁₈-n-Paraffine.
(4) Weitere geeignete anionische Tenside sind die Reaktionsprodukte von Fettsäuren, verestert mit Isothionsäure und neutralisiert mit Natriumhydroxid, worin die Fettsäuren beispielsweise aus Cocosnußöl abgeleitet sind; Natrium oder Kaliumsalze von Fettsäureamiden von Methyltaurid, worin die Fettsäuren beispielsweise aus Kokosnussöl abgeleitet sind.
(5) Mono-, Di- und Trialkylphosphosäureester und deren Alkoxylate (Ethoxylate, Propoxylate und Mischvarianten).
(6) Olefinsulfonate mit etwa 10 bis etwa 24 Kohlenstoffatomen, die durch Sulfonierung von alpha-Olefinen mittels unkomplexiertem Schwefeltrioxid gebildet sind, wobei die Säure-Reaktionsmischung so neutralisiert wurde, dass jegliche gebildete Sulfone, unter Bildung der entsprechenden Hydroxyalkansulfonate hydolysiert werden. Die Alpha-Olefine, aus denen die Olefinsulfonate abgeleitet werden, sind vorzugsweise geradkettige Mono-Olefine mit 12 bis 24 Kohlenstoffatomen, vorzugsweise 14 bis 16 Kohlenstoffatomen. Zusätzlich zu den echten Alkensulfonaten und einem Anteil Hydroxyalkansulfonaten können die Olefinsulfonate kleinere Mengen anderer Materialien enthalten, wie Alkendisulfonate, was von den Reaktionsbedingungen, dem Verhältnis der Reaktanten, der Natur der Ausgangsolefine und Verunreinigungen im Olefinausgangsmaterial und Nebenreaktionen während des Sulfonierungsverfahren abhängig ist
(7) Eine weitere Klasse der anionischen Tensiden sind die beta-Alkyloxyalkansulfonate. Diese Tenside weisen die folgende Formel auf: 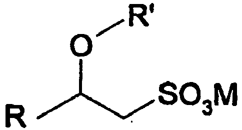 worin R eine geradkettige Alkylgruppe mit 6 bis 20 Kohlenstoffatomen ist, R' eine Niederalkylgruppe mit 1 (bevorzugt) bis 3 Kohlenstoffatomen ist, und M ein wasserlösliches Kation, wie hierin obenstehend beschrieben, ist. Als ethoxylierte Alkylethercarbonsäure bzw. deren Salz kann vorteilhaft das Natriumlaureth-11-carboxylat verwendet werden.
(8) Eine weitere geeignete Klasse von anionischen Tensiden sind anionische Geminitenside, die dadurch gekennzeichnet sind, dass zwei Tensideinheiten bestehend aus einer hydrophoben Gruppe und einer hydrophilen Gruppe durch einen Spacer nahe der hydrophilen Gruppe miteinander verbunden sind. In der Patentschrift DE 19943668 und DE 19505368 werden besonders geeignete Geminitenside ausführlich beschrieben. Ganz besonders geeignet sind die sulfatierten, carboxymethylierten und/oder phosphatierten und anschließend neutralisierten Derivate der Alkylen- N, N'-Diacyl-N, N'-dialkoxylate, sowie der Diacylen-N,N'-Dialkyl-N,N'-dialkoxylate.

### Amphotere und zwitterionische Tenside

Als geeignete amphotere Tenside seien genannt Alkylaminoalkancarbonsäuren, Betaine, Sulfobetaine und Imidazolinderivate. Amphotere Tenside schließen weiterhin die Derivate von aliphatischen sekundären und tertiären Aminen ein, in denen der aliphatische Rest linear oder verzweigt ist und worin einer der aliphatischen Substituenten 8 bis 18 Kohlenstoffatome enthält, und einer eine anionische Wasserlöslichkeit vermittelnde Gruppe, z. B. Carboxy, Sulfonat, Sulfat, Phosphat oder Phosphonat, enthält. Alkylamidoamphoacetate, Alkylamidoamphodiacetate sind ebenfalls geeignet. Zwitterionische Tenside, die Derivate von aliphatischen quaternären Ammonium-, Phosphonium- und Sulfoniumverbindungen einschießen, in denen die aliphatischen Reste linear oder verzweigt sein können und worin einer der aliphatischen Substituenten 8 bis 18 Kohlenstoffatome enthält, und einer eine anionische Gruppe, z. B. Carboxy, Sulfonat, Sulfat, Phosphat oder Phosphonat, enthält. Eine allgemeine Formel für diese Verbindungen ist 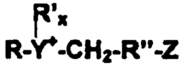 worin R einen Alkyl-, Alkenyl- oder Hydroxyalkylrest von 8 bis 18 Kohlenstoffatomen, 0 bis 10 Ethylenoxid-Gruppen und 0 bis 1 Glyceryleinheit(en) enthält; Y gewählt wird aus der Gruppe, bestehend aus Stickstoff-, Phosphor- und Schwefelatomen; R' eine Alkyl- oder Monohydroxyalkylgruppe mit 1 bis 3 Kohlenstoffatomen ist; X 1 ist, wenn Y ein Schwefelatom ist, und 2 ist, wenn Y ein Stickstoff- oder Phosphoratom ist; R" ein Alkylen oder Hydroxyalkylen mit 1 bis 4 Kohlenstoffatomen ist, und Z ein Rest ist, gewählt aus der Gruppe, bestehend aus Carboxylat-, Sulfonat-, Sulfat-, Phosphonat- und Phosphatgruppen.

Beispiele von amphoteren und zwitterionischen Tensiden schließen auch Sultaine und Amidosultaine ein. Sultaine und Amidosultaine können als schaumverstärkende Tenside, welche gegenüber dem Auge mild sind, in teilweisem Ersatz für die anionischen Tenside verwendet werden. Sultaine, einschließlich Amidosultainen, schließen z. B. Cocosdimethylpropylsultain, Stearyldimethylpropylsultain, Lauryl-bis-(2-hydroxyethyl)propylsultain und die Amidosultaine, z. B. Cocosamidodimethylpropylsultain, Stearylamidodimethylpropylsultain, Laurylamidobis-(2-hydroxyethyl)propylsultain ein. Bevorzugt werden Amidohydroxysultaine, wie die C₁₂-C₁₈-Hydrocarbylamidopropylhydroxysultaine speziell C₁₂-C₁₄-Hydrocarbylamidopropylhydroxysultaine, z. B. Laurylamidopropylhydroxysultaine und Cocamidopropylhydroxysultaine.

Weitere geeignete amphotere Tenside sind die Aminoalkanoate der Formel R-NH(CH₂)nCOOM, die Iminodialkanoate der Formel R-N[(CH₂)ₘCOOM]₂ und Mischungen hiervon; worin n und m Zahlen von 1 bis 4 sind, R C₈- bis C₂₂- Alkyl oder Alkenyl und M Wasserstoff, Alkalimetall, Erdalkalimetall, Ammonium oder Alkanolammonium ist.

Beispiele von geeigneten Aminoalkanoaten schließen n-Alkylaminopropionate und n-Alkyliminodipropionate ein, wofür Beispiele N-Lauryl-beta-aminopropionsäure oder deren Salze und N-Lauryl-beta-imino-dipropionsäure oder deren Salze sind. Andere geeignete amphotere Tenside sind gekennzeichnet durch die Formel: 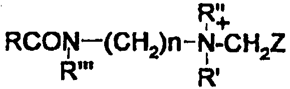 worin R C₈- - C₂₂- Alkyl oder -Alkenyl, vorzugsweise C₁₂ - C₁₆ ist, R' Wasserstoff oder CH₂CO₂M ist, R" CH₂CH₂OH oder CH₂CH₂OCH₂CH₂COOM ist, R'" Wasserstoff, CH₂CH₂OH oder CH₂CH₂OCH₂CH₂COOM ist, Z CO₂M oder CH₂CO₂M ist, n 2 oder 3, vorzugsweise 2 ist, M Wasserstoff oder ein Kation ist, wie Alkalimetall (z. B. Lithium, Natrium, Kalium), Erdalkalimetall (Beryllium, Magnesium, Calcium, Strontium, Barium) oder Ammonium.

Beispiele von Tensiden der obenstehenden Formel sind Monocarboxylate und Dicarboxylate. Geeignete Beispiele schließen Cocoamphocarboxypropionat, Cocoamphocarboxypropionsäure, Cocoamphocarboxyglycinat (alternativ als Cocoamphodiacetat bezeichnet) und Cocoamphoacetat ein.

Handelsübliche amphotere Tenside schließen diejenigen ein, welche unter den Handelsnamen MIRANOL C2M CONC. N.P., MIRANOL C2M CONC. O.P., MIRANOL C2M SF, MIRANOL CM SPECIAL (Miranol, Inc.); ALKATERIC 2CIB (Alkaril Chemicals); AMPHOTERGE W-2 (Lonza, Inc.); MONATERIC CDX-38, MONATERIC CSH-32 (Mona Industries); REWOTERIC AM-2C (Rewo Chemical Group) und SCHERCOTERIC MS-2 (Scher Chemicals) vertrieben werden.

Geeignete zwitterionische (Betain-)Tenside sind z.B. solche die durch nachstehende Formel repräsentiert sind (die Formel müssen zu den Bezeichnungen passen): 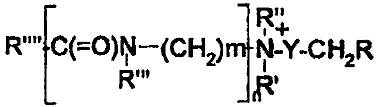 worin
- R: COOM oder CH(OH)-CH₂SO₃M ist und
- R': ein Niederalkyl. oder Hydroxyalkyl ist,
- R'': ein Niederalkyl oder Hydroxyalkyl ist,
- R'": ein Vertreter ist, gewählt aus der Gruppe, bestehend aus Wasserstoff und Niederalkyl,
- R"": ein höheres Alkyl oder Alkenyl ist,
- Y: ein Niederalkyl, vorzugsweise Methyl ist,
- m: eine ganze Zahl von 2 bis 7, vorzugsweise von 2 bis 3 ist,
- n: die ganze Zahl 1 oder 0 ist, und
- M: Wasserstoff oder ein Kation, wie oben beschrieben, z. B. ein Alkalimetall, Erdalkalimetall oder Ammonium, ist.

Der Begriff "Niederalkyl" oder "Hydroxyalkyl" bedeutet lineare oder verzweigte, gesättigte aliphatische Kohlenwasserstoffreste und substituierte Kohlenwasserstoffreste mit einem bis etwa drei Kohlenstoffatomen, wie beispielsweise Methyl, Ethyl, Propyl, Isopropyl, Hydroxypropyl, Hydroxyethyl und dergleichen. Der Begriff "höheres Alkyl oder Alkenyl" bedeutet lineare oder verzweigte, gesättigte (d. h. "Höheralkyl") und ungesättigte (d. h. "Höheralkenyl") aliphatische Kohlenwasserstoffreste mit acht bis 20 Kohlenstoffatomen, wie zum Beispiel Lauryl, Cetyl, Stearyl, Oleyl. Der Begriff "höheres Alkyl oder Alkenyl" schließt Mischungen von Resten ein, welche eine oder mehrere intermediäre Bindungen wie Ether- oder Polyether-Bindungen, oder nicht-funktionelle Substituenten, wie Hydroxyl- oder Halogenreste, enthalten können, wobei der Rest von hydrophobem Charakter bleibt.

Beispiel von Betain-Tensiden der obenstehenden Formel, worin n Null ist, schließen die Alkylbetaine, wie Cocosdimethylcarboxymethylbetain, Lauryldimethylcarboxymethylbetain, Lauryldimethyl-alpha-carboxyethylbetain, Cetyldimethylcarboxymethylbetain, Lauryl-bis-(2-hydroxyethyl)carboxymethylbetain, Stearyl-bis(2-hydroxypropyl)carboxymethylbetain, Oleyldimethyl-gamma-carboxypropylbetain, Lauryl-bis(2-hydroxypropyl)-alpha-carboxyethylbetain ein. Die Sulfobetaine können repräsentiert werden durch Cocosdimethylsulfopropylbetain, Stearyldimethylsulfopropylbetain und Lauryl-bis(2-hydroxyethyl)sulfopropylbetain.

Spezifische Beispiele sind Amidobetaine und Amidosulfobetaine umfassend die Amidocarboxybetaine, wie Cocosamidodimethylcarboxymethylbetain, Laurylamidodimethylcarboxymethylbetain, Cetylamidodimethylcarboxymethylbetain, Laurylamido-bis-(2-hydroxyethyl)carboxymethylbetain und Cocosamido-bis-(2-hydroxyethyl)carboxymethylbetain.

Die Amidosulfobetaine können durch Cocosamidodimethylsulfopropylbetain, Stearylamidodimethylsulfopropylbetain und Laurylamido-bis(2-hydroxyethyl)sulfo-propylbetain repräsentiert werden.

Nachfolgend seien Beispiele für geeignete kationische Tenside aufgeführt:

Genannt sind z.B. quaternäre Ammoniumverbindungen entsprechend der allgemeinen Formel: 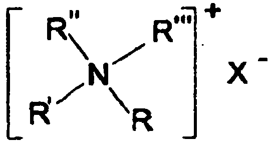 worin R, R', R" und R'" unabhängig eine aliphatische Gruppe von 1 bis 22 Kohlenstoffatomen, oder eine aromatische, Alkoxy-, Polyoxyalkylen-, Alkylamido-, Hydroxyalkyl-, Aryl- oder Alkylaryl-Gruppe mit bis zu 32 Kohlenstoffatomen sind und X ein salzbildendes Anion wie Halogen (zum Beispiel Chlorid, Bromid), Acetat-, Citrat-, Lactat-, Glycolat-, Phosphat-, Nitrat-, Sulfat-, Methosulfat und Alkylsulfat-Resten ist. Die aliphatischen Gruppen können, zusätzlich zu Kohlenstoff- und Wasserstoff-atomen, Ether- und andere Gruppen, wie Aminogruppen, enthalten. Die längerkettigen aliphatischen Gruppen, mit z. B. 12 Kohlenstoffatomen oder mehr, können gesättigt oder ungesättigt sein.

Es ist bevorzugt, wenn R, R', R" und R'" unabhängig voneinander C₁- bis C₂₂-Alkyl sind. Insbesondere bevorzugt sind derartige Verbindungen mit zwei langen Alkylketten und zwei kurzen Alkylketten oder einer langen Alkylkette und drei kurzen Alkylketten.

Die langen Alkylketten haben 12 bis 22 Kohlenstoffatome, vorzugsweise 16 bis 22 Kohlenstoffatome, und die kurzen Alkylketten weisen 1 bis 4 Kohlenstoffatome, vorzugsweise 1 bis 3 Kohlenstoffatome, auf. Darüber hinaus sind geeignet Alkyl-Pyridiniumsalze, Salze von Aminoxiden, Sulfoniumsalze und Tropyliumsalze. Besonders geeignet sind Cetyl-, Cetearyl- und Behenyl- Trimethylammonium- Choride, Bromide und Methosulfate (INCI).

Als besonders geeignet einzusetzende nichtionische oder anionische Tenside seien folgende Geminitenside genannt, wobei bei unterschiedlichen Alkoxylat - Gruppen die Alkoxylat - Gruppen jeweils statistisch verteilt oder in Blockstruktur angeordnet sein können. Bei Blockstruktur-Anordnung ist es besonders vorteilhaft ist, wenn zuerst der Propoxylat - Block verknüpft wird.

Besonders geeignet sind die Strukturen A.I, A.II und B.III.

Auf amid- oder aminhaltigen Spacem beruhende Strukturen
A.I Geminitenside der allgemeinen Formel (A.I) analog WO 96/14926 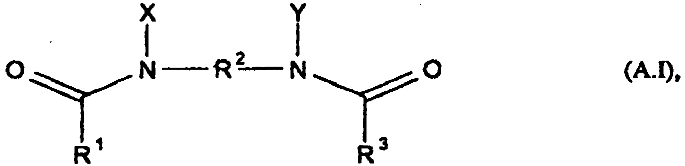 wobei die Substituenten folgende Bedeutung haben:
   - R¹, R³: C₅- bis C₂₅-Alkyl, verzweigt oder unverzweigt, auch ungesättigt;
   - R²: C₁- bis C₁₂-Alkylen;
   - X, Y: (C₂H₄O-)ₓ(C₃H₆O-)_{y}-FR ; x+y ≥ 1, x: 0-15, y: 0-10 und
   - FR: -SO₃M, -CH₂-CO₂M, -P(O)(OM)₂, H, -C₃H₆SO₃M; oder -CH₂(CHOH)₄CH₂OH, soweit x+y=0; wobei M = Alkali, (Alkyl)Ammonium, Alkanolammonium, H oder ½ Erdalkali ist.
A.II Geminitenside mit Dicarbonsäure-stämmigen Spacern der allgemeinen Formel (A.II) analog WO 96/25388 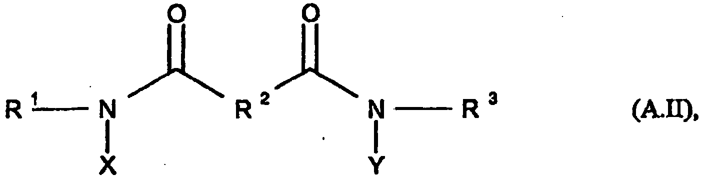 wobei die Substituenten die für die allgemeine Formel (A.I) angegebene Bedeutung haben.
A.III Amphotere Geminitenside der allgemeinen Formel (A.III) analog WO 97/31890 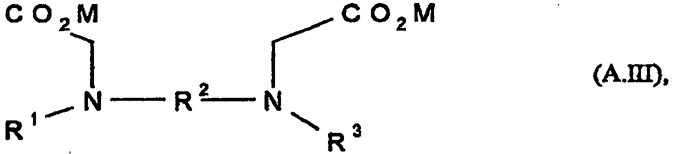 wobei die Substituenten die für die allgemeine Formel (A.I) angegebene Bedeutung haben. Geminitenside der allgemeinen Formel (A.III) sind amphotere Verbindungen, so daß sie bei entsprechend saurem Umgebungsmedium auch kationisch werden können.

Auf amid- oder aminhaltigen Spacern beruhende Strukturen
B.I Geminitenside der allgemeinen Formel (B.I) analog DE 19622612 oder JP-A 10-175934 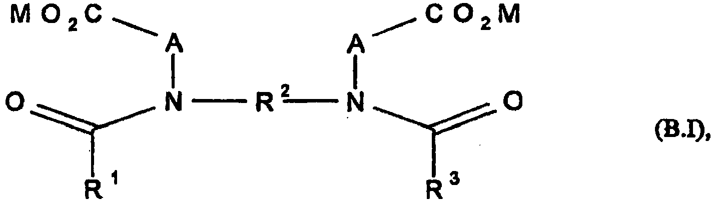 wobei die Substituenten folgende Bedeutung haben:
   - R¹, R³: C₅- bis C₂₅- Alkyl, verzweigt oder unverzweigt, auch ungesättigt;
   - R²: C₁- bis C₁₂-Alkylen;
   - A: CHR⁴, CH₂, C₂H₄, C₃H₆, C₄H₈;
   - R⁴: Rest einer Aminocarbonsäure und
   - M: Alkali, (Alkyl)Ammonium, Alkanolammonium, H oder ½ Erdalkali.
B.II Geminitenside der allgemeinen Formel (B.II) analog EP 0 708 079 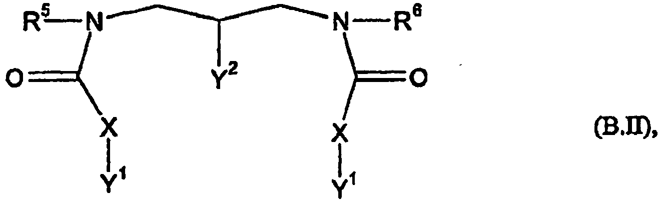 wobei die Substituenten die für die allgemeine Formel (B.I) angegebene Bedeutung haben und
   - R⁵, R⁶: C₆- bis C₃₆-Alkyl, verzweigt oder unverzweigt, auch ungesättigt;
   - X: Alkylen- oder Alkenylengruppe mit 1 bis 6 Kohlenstoffatomen, die mit einer Hydroxylgruppe oder einer Sulfonsäuregruppe oder einer Carboxygruppe substituiert sein kann;
   - Y¹: eine Sulfonat- oder Sulfatgruppe oder eine Carboxylgrupppe und
   - Y²: eine Hydroxylgruppe, ein Schwefelsäurerest oder -O-(CO)X-COOH bedeuten.
B.III Geminitenside der allgemeinen Formel (B.III) analog JP-A-8-311003 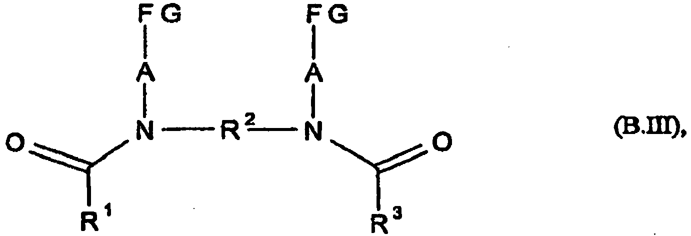 wobei die Substituenten die für die allgemeine Formel (B.I) angegebene Bedeutung haben und
   - FG: -COOM oder -S0₃M bedeutet
B.IV Geminitenside der allgemeinen Formel (B.IV) analog JP-A11-60437 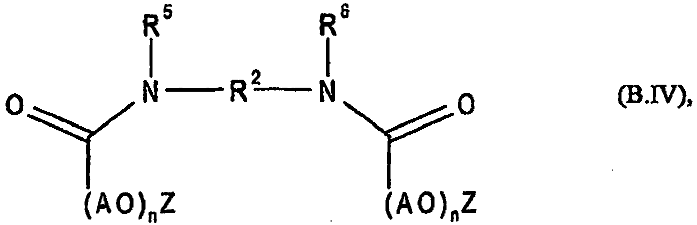 wobei die Substituenten, die bei den allgemeinen Formeln (B.I) und (B.II) angegebene Bedeutung haben und
   - AO: Alkylenoxideinheiten, d.h. Ethylenglykol-, Propylenglykol und Butylenglykolethereinheiten, allein oder statistisch oder blockweise verteilt, mit n = 1 bis 20 und
   - Z: -SO₃M, -C₂H₄SO₃M, -C₃H₆SO₃M, -P(O)(OM)₂ ober -CH₂-COOM, -C₂H₄-COOM bedeuten.

Auf amid- oder aminhaltigen Spacern beruhende Strukturen
C.I Geminitenside der allgemeinen Formel (C.I) analog EP 0 697 244 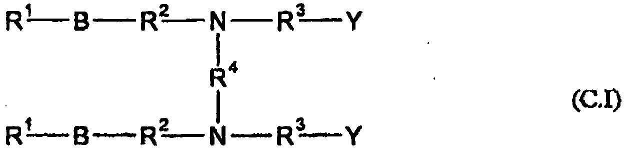 wobei die Substituenten folgende Bedeutung haben:
   - R¹: C₅- bis C₂₅-Alkyl, verzweigt oder unverzweigt, auch ungesättigt, hydroxysubstituiert oder perfluoriert;
   - R²: C₁- bis C₁₂-Alkylen oder hydroxysubstituierte Derivate davon;
   - B: eine Amidgruppe [-C(O)N(R²)- oder -N(R⁵)C(O)-], eine Carboxylgruppe [-C(O)O- oder -OC(O)-], eine Polyethergruppe [-(O(R⁶-O)ₖ-];
   - R⁵: für C₁- bis C₄-Alkyl oder hydroxysubstituiertes Alkyl oder H steht;
   - R⁶: für C₂- bis C₄-Alkylen;
   - x: eine Zahl von 1 bis 20;
   - R³: für C₁- bis C₁₂- Alkyl oder hydroxysubstituierte Derivate davon, R⁷-D-R⁷ oder eine Polyethergruppe [-(O(R⁶-O)ₓ-];
   - R⁷: für C₁- bis C₆- Alkylen oder hydroxysubstituierte Derivate davon;
   - D: -O-, -S-, -N(R⁸)- ;
   - R⁴: Alkylen oder Alkylaryl mit 1 bis 12 C-Atomen oder die hydroxysubstituierten Derivaten oder R⁹-D¹-R⁹ ;
   - R⁸: C₁- bis C₁₂-Alkyl oder hydroxysubstituiertes Alkyl oder H oder R⁹-D¹-R⁹;
   - R⁹: for C₁- bis C₆- Alkylen oder hydroxysubstituierte Derivate davon oder Aryl;
   - D¹: -O-, -S-, -SO₂-, -C(O)-, [-(O(R⁷-O)ₓ-], (R¹⁰)ₜ[N(R¹⁰)]_{z} oder Aryl;
   - R¹⁰: C₁- bis C₁₂-Alkyl oder hydroxysubstituiertes Alkyl oder H oder Aryl;
   - t,z: unabhängig voneinander eine Zahl von 1 bis 4 bedeuten und
   - Y: unabhängig voneinander für -SO₃H, O-SO₃H, -OP(O)(OH)₂, -P(O)(OH)₂, -COOH, -CO₂-C₆H₄-SO₃H und deren Salze davon steht.
C.II Geminitenside der allgemeinen Formel (C.II) analog EP 0 697 245 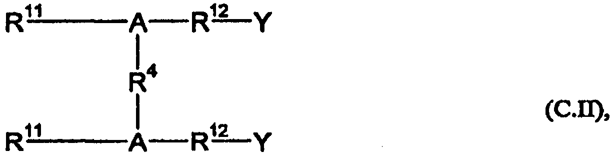 wobei die Substituenten die für die allgemeine Formel (C.I) angegebene Bedeutung haben und
   - R¹¹: C₅- bis C₂₃-Alkyl, verzweigt oder unverzweigt, auch ungesättigt, hydroxysubstituiert oder perfluoriert oder R¹⁴-B-R²;
   - R¹⁴: C₁- bis C₁₂-Alkyl, verzweigt oder unverzweigt, auch ungesättigt, oder die hydroxysubstituierten Derivate;
   - R¹²: C₁- bis C₁₂-Alkylen, verzweigt oder unverzweigt, gesättigt, gegebenenfalls bis zu 2-fach nicht-benachbart ungesättigt, oder die hydroxysubstituierten Derivate oder eine Amidgruppe [-C(O)N(R²)- oder -N(R⁵)C(O)-], eine Carboxylgruppe [-C(O)O- oder-OC(O)-], eine Polyethergruppe [-(O(R⁶-O)ₓ-] oder R⁹-D^{I}-R⁹ und
   - A: -CR⁶= oder -N= unter der Voraussetzung, daß wenn A gleich -N= ist, R¹¹ gleich R¹⁴-B-R² bedeuten.
C.III Geminitenside der allgemeinen Formel (C.III) analog DE 4227391 und DE 19608117 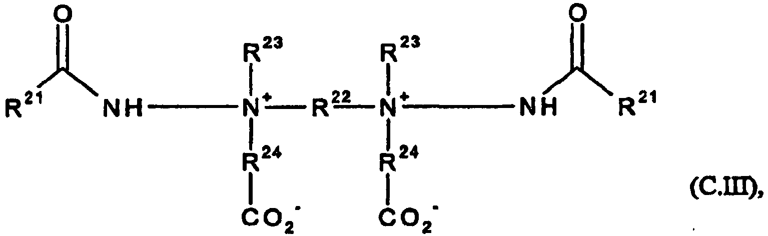 wobei die Substituenten die für die allgemeinen Formeln (C.I) und (C.II) angegebene Bedeutung haben und
   - R²¹: C₅- bis C₂₃-Alkyl, verzweigt oder unverzweigt, auch ungesättigt;
   - R²², R²⁴: C₁- bis C₆ Alkylen;
   - R²³: Methyl, Ethyl, Propyl oder eine Polyethergruppe [-(O(R⁶-O)ₓ-]
   bedeuten.
D. I Geminitenside der allgemeinen Formel (D.I) analog US 5,863,886 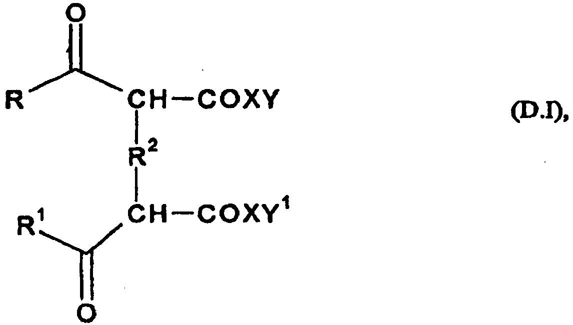 wobei die Substituenten folgende Bedeutung haben:
   - R, R¹: C₅- bis C₃₀-Alkyl, verzweigt oder unverzweigt, auch ungesättigt, hydroxy-substituiert oder perfluoriert;
   - R²: C₁-bis C₁₀- Alkylen, Arylen und hydroxysubstituierte Derivate, ein Polyether [-O(R⁴O)ₓ-], -S-, -SO₂-, -O-, -S-S-, -O-R⁵-O- oder -S-R⁵-S-; Variable für eine direkte Bindung zwischen den beiden α-Kohlenstoffen;
   - R⁴: C₂- bis C₄-Alkylen;
   - R⁵: C₁- bis C₁₀-Alkylen, Arylen oder Alkylarylen, -N(R⁶)- oder -(NR⁶)-R⁷-(NR⁶)-;
   - R⁶: C₁- bis C₆-Alkyl;
   - R⁷: C₁- bis C₆-Alkyl, wobei R⁷ und R⁶ auch Teil eines heterocyclischen Ringes sein können;
   - X: Polyether [-O(R⁴O)ₓ-], wobei x eine Zahl von 1 bis 30 ist, -O-, NZ;
   - Z: C₁- bis C₁₀- Alkyl, Aryl, Alkylaryl oder H und
   - Y, Y¹: unabhängig voneinander H, -CH₂-COOH und Salze, ein Kohlenwasserstoffrest mit mindestens 2 Hydroxylgruppen, wie Erythrose, Threose, Ribose, Arabinose, Xylose, Fructose, Lyxose, Allose, Altrose, Glucose, Mannose, Galactose und ihre Mischungen.
D.II Geminitenside, der allgemeinen Formel (D.II) 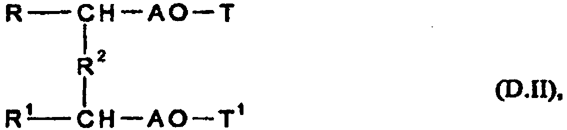 wobei die Substituenten die für die allgemeine Formel (D.I) angegebene Bedeutung haben und
   - AO: -C(O)-, -C(O)- [-O(R⁴O)ₓ-], -CH₂- [-O(R⁴O)ₓ-], -CH₂-O-;
   - T, T¹: unabhängig voneinander -OM, -H, -CH₃, -C₂H₅, -SO₃M, -CH₂COOM, -C₂H₄-COOM, -C₃H₆-SO₃M, -O-P(O)(OM)₂ und
   - M: Alkali, ½ Erdalkali, Ammonium, Mono-, Di-, Trialkanolammonium oder H bedeuten.
D.III Geminitenside der allgemeinen Formel (D.III) analog WO 96/16930 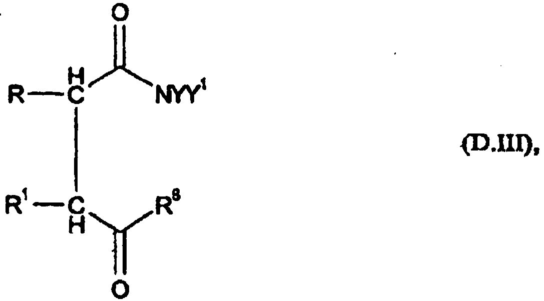 wobei die Substituenten die für die allgemeinen Formeln (D.I) und (D.II) angegebene Bedeutung haben und
   - R⁸: NYY¹, -O(R⁴O)ₓH oder -O(R⁴O)ₓ-C(O)-CHR-CHR¹-C(O)NYY¹ bedeutet
D.IV Geminitenside der allgemeinen Formel (D.IV) analog WO 96/25384 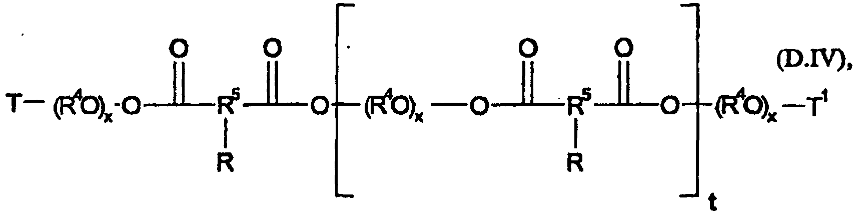 wobei die Substituenten die für die allgemeinen Formeln (D.I), (D.II) und (D.III) angegebene Bedeutung haben und
   - t: eine ganze Zahl von 1 bis 100, bevorzugt 1 bis 20, besonders bevorzugt 1 bis 4 bedeutet.

### Ölphase

Die Ölphase der O/W-Emulsion der erfindungsgemäßen Formulierungen wird vorteilhaft gewählt aus der Gruppe der polaren Öle, beispielsweise aus der Gruppe der Fettsäuretriglyceride, namentlich Glycerin Triester gesättigter und/oder ungesättigter, verzweigter und/oder unverzweigter Alkancarbonsäuren einer Kettenlänge von 8 bis 32, insbesondere 12 bis 18 C-Atomen.

Die Fettsäuretriglyceride können beispielsweise vorteilhaft gewählt werden aus der Gruppe der synthetischen, halb- synthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Babassuöl, Karottenöl, Palmkernöl und dergleichen mehr.

Darüber hinaus können erfindungsgemäß eingesetzt werden synthetisch hergestellte Ester von C6- bis C32- Carbonsäuren, oder Hydroxycarbonsäuren linear oder verzweigt, gesättigt oder ein bis dreifach ungesättigt, mit Di-, Tri- oder Polyhydroxyverbindungen, die alle mit Carbonsäuren abgesättigt sein können oder nur zu einem Teil, mindestens aber einfach umgesetzt sein müssen und keine signifikante Grenzflächenaktivität aufweisen dürfen. Besonders geeignet sind hier die MCT Öle (MCT = Mid-chain triglyceride) wie Caprylic/Capric Triglyceride oder auch Butylenglycol Dicaprylate / Dicaprate und die entsprechende Propylenglykolvarianten aber auch entsprechende Ester des Propylen- und Butylenglykols. Weiter eignen sich (INCI) Bis-Diglyceryl-Polyacyl-adipate-1 und -2 ebenso wie ihre Vorbilder aus der Natur, Lanolin und Lanolinöl sowie Lanolinalkohol.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 40 C-Atomen und gesättigten und/oder ungesättigten, verzweigten und/oder unverzweigten Alkoholen einer Kettenlänge von 3 bis 40 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 40 C-Atomen.

Esteröle können dann vorteilhaft gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethyl-hexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl, Mandel-, Orangen-, Macadamia-, Babassu-, Evening Primrose und andere Öle. Guerbetalkohole von C10 bis C36 können ebenfalls als polare Ölkomponenten eingesetzt werden.

Ferner können die Ölphasen vorteilhaft gewählt werden aus der Gruppe der Dialkylether und Dialkylcarbonate, vorteilhaft sind z. B. Dicaprylylether (Cetiol OE, Cosmacol OE) und/oder Dicaprylylcarbonat, beispielsweise das unter der Handelsbezeichnung Cetiol CC bei der Fa. Cognis erhältliche.

Die Ölkomponente kann ferner sein ein Neopentylglykoldiheptanoat, ein Propylenglykoldicaprylat/dicaprat, ein Caprylic/Capric/Diglycerylsuccinat, ein Butylenglykol-Dicaprylat/Dicaprat, ein C12/13-Alkyllactat, ein Di-C12/13-Alkyltartrat, ein Triisostearin, ein Dipentaerythrityl-Hexacaprylat/Hexacaprat, Propylenglykolmonoisostearat, und/oder Tricaprylin.

Vorteilhafte ölkomponenten sind ferner z.B. Butyloctylsalicylat (beispielsweise das unter der Handelsbezeichnung Hallbrite BHB bei der Fa. CP Hall erhältliche), Hexadecylbenzoat und Butyloctylbenzoat und Gemische davon (Hallstar AB) und/oder Diethylhexylnaphthalat.

Auch beliebige Abmischungen solcher Ölkomponenten sind vorteilhaft im Sinne der vorliegenden Erfindung einzusetzen.

Ferner können die Ölphasen ebenfalls vorteilhaft auch unpolare Öle enthalten, beispielsweise verzweigte und unverzweigte Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft können die Ölphasen ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten einzusetzen. Silikonöle sind synthetische polymere Verbindungen, in denen Silicium-Atome über Sauerstoff-Atome verknüpft sind. Die methylsubstituierten Polyorganosiloxane werden auch als Polydimethylsiloxan bzw. Dimethicon (INCI) bezeichnet. Dimethicone gibt es in verschiedenen Kettenlängen bzw. mit verschiedenen Molekulargewichten. Ebenso verwendbar sind (INCI) Dimethiconole.

Besonders vorteilhafte Polyorganosiloxane im Sinne der vorliegenden Erfindung sind beispielsweise Dimethylpolysiloxane [Poly(dimethylsiloxan)]. Ferner vorteilhaft sind Phenylmethylpolysiloxane (INCI: Phenyl Dimethicone, Phenyl Trimethicone), cyclische Silikone (Decamethylcyclopentasiloxan, Dodecamethylcyclohexasiloxan), welche nach INCI auch als Cyclomethicone bezeichnet werden, aminomodifizierte Silikone (INCI: Aminodimethicone) und Silikonwachse, z. B. Polysiloxan-PolyalkylenCopolymere (INCI : Stearyl Dimethicone und Cetyl Dimethicone) und Dialkoxydimethylpolysiloxane (Stearoxy Dimethicone und Behenoxy Stearyl Dimethicone).

Bitumenmischungen verschiedener Viskositäten und Provinienzen (aus Petrochemie oder aus natürlich vorkommenden Quellen (sog. Asphaltene) können ebenfalls vorteilhaft eingesetzt werden. Bei zu hohen Erweichungspunkten muss in Drucksystemen und oberhalb von 100 °C gearbeitet werden, was die erfindungsgemäße Verwendung aber nicht einschränkt. In diesem Fall müssen besonders hitzeresistente Tenside eingesetzt werden.

Es kann auch gegebenenfalls vorteilhaft sein, Wachse, beispielsweise Cetylpalmitat, als alleinige Lipidkomponente der Ölphase einzusetzen. Vorteilhaft wird die Ölphase gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether, didodecylether, didecylether.

Ferner können Perfluoroether, Perflouroalkohole und - säuren und deren Derivate, sowie mit perfluoro-Gruppen derivatisierte Silicone und - Silane alleinig oder als Bestandteile der Ölphase eingesetzt werden.

Ferner können vorteilhaft in der Ölphase bzw. als alleinige Komponenten der Ölphase eingesetzt werden Ester von Hydroxycarbonsäuren, besonders bevorzugt sind Alkylcarbon- und Benzoyl- wie auch Salicylsäureester mit Alkoholen, mit C2 bis C40, linear oder verzweigt, gesättigt oder ungesättigt und Mischungen der verschiedenen Alkohole. Die Alkylester der Glykolsäure, Milchsäure, Zitronensäure, Weinsäure, Benzoesäure, 2-Ethylhexansäure, Apfel- und Äpfelsäure, Salicylsäure und Benzoesäure sind besonders geeignet.

Ferner kann die Ölphase auch aus Monomeren und deren Polymeren und CoPolymeren bestehen, Vinylakohol und seine Ester, bevorzugt Vinylacetat, Vinylchlorid, Acrylsäure und deren Ester, Methacrylsäure und deren Ester, Ethylen, alpha-und mittelständige Olefine mit C2 bis C22, besonders geeignet Ethylen, Propylen, Butylen, Styrol, Cyclopentadien und/oder Butadien.

Die Viskosität der aus der Verdünnung des selbstemulgierenden Gels (G) entstehenden Nanoemulsion (C) kann mit Hilfe aller gängigen Hydrokolloide und Verdicker kontrolliert werden.

Diese Hydrokolloide und Verdicker können vorteilhaft gewählt werden aus der Gruppe der organischen oder anorganischen Verdicker und Hydrokolloide. Unter den organischen Hydrokolloiden sind sowohl die neutralen, anionischen als auch kationischen und amphoteren geeignet, wobei sowohl lineare als auch Cross Polymere als Hydrokolloide und Verdicker sich als geeignet für die erfindungsgemäßen Emulsionen erwiesen haben. Unter den anorganischen sind sowohl die reinen anorganischen Verdicker wie auch die organisch modifizierten Verdicker und Hydrokolloide geeignet.

Darüber hinaus können synergistisch wirkende Kombinationen innerhalb beider Gruppen wie auch aus beiden Gruppen stammend günstig eingesetzt werden.

Als Beispiele sollen hier Assoziativverdicker wie hydrophob modifizierte Acryl-, Metacryl, Maleinsäure- homo- und / oder Copolymerisate, Polyurethanderivate, Acrylamidderivate, Non-Assoziatiwerdicker wie quellbare Verdicker basierend auf Acryl-, Methacryl, Maleinsäure Homo- oder Copolymer und deren Derivaten, Cellulose und Cellulosederivate (Carboxymethylcellulose, Hydroxyethylcellulose, Methylcellulose, Methylhydroxyethylcellulose, Methylhydroxypropylcellulose, Ethylhydroxyethylcellulose, hydrophob modifizierte Ethylhydroxyethylcellulose, mikrokristalline Cellulose, Cellulose Gum und Mischungen aus diesen beiden mit unterschiedlichen Verhältnissen von mikrokristalliner Cellulose zu Cellulose Gum, hydrophob modifizierte Hydroxyethylcellulose), Tone (Schichtsilicate, Smectite, Alumosilicate oder Schichtsilicate wie Montmorillonit, Bentonit Hectorit, Attapulgite) auch Magnesium Aluminium Silicate und Organotonderivate (organisch modifizierte Alumosilicate, Bentonite, Hectorite) aber auch Kieselsäurederivate (Fumed Silica), Organowachse (Rhizinusölderivate, Polyamidbasierte Organowachse), Polyesteramide und metallorganische Verdicker (Titanate und Zirconate) - besonders in Kombination mit Stärke- und Cellulosederivaten, natürliche Gum-Derivate wie Guar- oder Stärkederivate, genannt sein.

In der Gruppe der Gumme sind besonders geeignet die natürlichen und die modifizierten Polymere, bevorzugt pflanzlichen Ursprungs wie Gummi Arabicum, das aus verschienen Akazienarten hergestellt werden kann. Auch von anderen Bäumen lassen sich natürliche Gummen gewinnen, z.B. Tragacanth Gum. Ebenfalls geeignet sind Alginate und Carrageenan und ihre Derivate. Xanthan Gum und seine Variationen und Derivate sind ebenfalls besonders als Hydrocolloid und Verdicker geeignet. Guar Caroube und Pectine gehören ebenfalls zur Gruppe der Gumme und sind geeignet.

Zu den natürlichen, als Verdicker und Hydrocolloid geeigneten Polymeren gehört ebenfalls die Stärke und ihre Derivate (Dextrinderivate), wie Carboxymethylstärke, Hydroxymethylstärke aber auch phosphatierte Stärke.

Elektrolyte, seien es ein- oder mehrwertige, werden bis zu einer Konzentration von ca. 40 %, bevorzugt bis 20 % und besonders bevorzugt bis 15 %, ohne signifikante Beeinträchtigung des Selbstemulgiermechanismusses und der daraus entstehenden Emulsionen toleriert.

Erfindungsgemässe kosmetische und dermatologische Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchhaltende Substanzen, organische Lösemittel, Aktivstoffe, wie zum Beispiel Vitamine und ihre Derivate, Pflanzenextrakte, Enzyme, Steroide und ihre Derivate und/oder Ceramide und ihre Derivate.

Besonders bevorzugte Anwendungen sind folgende:
- Emulsionen für Personal Care und Kosmetik
- Lebensmittelanwendungen
- Textil- und Lederhilfsmittel
- Emulsionen für den Agro-Bereich
- Emulsionen für die Metallbearbeitung
- Siliconölemulsionen in Wasser
- Emulsionen für die Bauchemie
- Emulsionen für die Haushalts- und Kfz-Pflege (Möbelpflege, Fahrzeuge aussen und innen)
- Emulsionen für die Schuh- und Textilpflege
- Emulsionen für die Herstellung von technischen Textilien
- Emulsionen von Fluorkohlenwasserstoffen und -derivaten
- Emulsionen von Polymeren für Versiegelungs-, Lack- und Klebstoffanwendungen
- Emulsionen von Polymeren für Non-Wovens, Wand- und sonstige Oberflächenbeschichtungen

Die nachfolgenden Beispiele illustrieren die Erfindung (% = Gew.%):

### Beispiel 1: Emulsionsbasis für z.B. Personal Care Anwendungen

| | | |
|---|---|---|
| A) | Imwitor 560 (Na- Lauroyl- Lactylat) | 1,9% |
| | Emuldac AS 80 (C16-18 -alcohol polyethyelene glycolether (>50 EO)) | 0,9% |
| | Glycerin | 1,8% |
| | Wasser (Aqua), Deionized | 2,7% |
| B) | MIGLYOL® 812 (Caprylic/Capric Triglyceride) | 41,6% |
| C) | Wasser (Aqua), Deionized | 51,1% |

### Herstellung:

Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße (D50, ermittelt aus statischer Laserlichtstreuung gemäß DIN/ISO 13320) von 400 nm auf.

### Beispiel 2: Personal Care Basic Milk

| | | |
|---|---|---|
| A) | IMWITOR 560 (Na-Lauroyl-Lactate) | 0.5% |
| | MARLIPAL 013/120 (Trideceth-12) | 0.25% |
| | Glycerin | 0.33% |
| | Wasser (Aqua), Deionized | 0.92% |
| B) | MIGLYOL® 8810 (Butylene Glycol Dicaprylate/Dicaprate) | 1.5% |
| | COSMACOL® EMI (C12-13 Alkyl Malate) | 1.0% |
| | DYNACERIN® 660 (Oleyl Erucate) | 1.0% |
| | Mineral Oil (Paraffinium Liquidium) | 6.5% |
| C) | Wasser (Aqua), Deionized | 73.8% |
| | Keltrol (Xanthan Gum) | 0.3% |
| | Glycerin | 2.0% |
| D) | Carbopol Ultrez 21 Polymer (3% ig)(Carbomer) | 10.0% |
| E) | NaOH (10%ig) | 1.2% |
| F) | Phenonip (Phenoxyethanol, Methylparaben, Ethylparaben, Butylparaben, Propylparaben und Isobutylparaben) | 0.7% |

Herstellung: Phase A homogen mischen. Phase B homogen mischen. Phase A vorlegen, Phase B unter Rühren im laminaren Strömungsfeld (ca. 1500 U/ min; Drahtrührer) ) langsam zudosieren, 1 min nachrühren. Phase C mischen, unter Rühren zugeben. Phase D unter Rühren zugeben, Phase E unter Rühren zugeben, Phase F unter Rühren zugeben. Die Emulsion weist eine mittlere Tröpfchengröße von 340 nm auf.

### Beispiel 3: Emulsion für personal care wet wipes

| | | |
|---|---|---|
| A) | IMWITOR 380 (Glyceryl Cocoate/Citrate/Lactate) | 2,0% |
| | Glucopon 215 CS UP (Capryl Glucoside) | 1,0% |
| | Decaglyn 1-L (Polyglyceryl-10 Laurate) | 0,3% |
| | Glycerin | 1,3% |
| | Wasser (Aqua), Deionized | 3,2% |
| B) | MIGLYOL® 812 (Caprylic/Capric Triglyceride) | 29,7% |
| C) | Wasser (Aqua), Deionized | 62,5% |

Herstellung: Phase A homogen mischen. Phase A vorliegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße von 400 nm auf:

### Beispiel 4: Kosmetische O/W Lotion

| | | |
|---|---|---|
| A) | AMPHOLYT JB 130 K (Cocoamidopropyl Betaine) | 2,7% |
| | Glycerin | 3,8% |
| B) | MIGLYOL® 812 (Caprylic/Capric Triglyceride) | 4,0% |
| | Dicapryl ether | 6,3% |
| | Cetearyl isononanoate | 3,0% |
| | Isopropylpalmitate | 8,0% |
| | Cyclomethicone | 2,0% |
| C) | Wasser (Aqua), Deionized | 20,0% |
| | Carbomer | 0,3% |
| D) | Wasser (Aqua), Deionized | 48,8% |
| | TEA | 0,2% |
| | Panthenol | 0,3% |
| | Phenonip | 0,7% |

Herstellung: Zuerst wird die Phase C hergestellt. Hierbei wird das Carbomer unter Rühren vollständig gequollen. Phasen A und B werden jeweils homogen gemischt und auf 70°C erwärmt Phase B wird unter Rühren im laminaren Strömungsfeld langsam der Phase A zugeführt Nach Zugabe wird je nach Ansatzgröße unter Rühren (laminare Fließbedingungen müssen erhalten bleiben) kurz homogenisiert. Danach werden die Phasen C und D nacheinander eingerührt. Die Emulsion weist eine mittlere Tröpfchengröße von 700 nm auf.

### Beispiel 5: Kosmetische O/W Lotion

### (auch Natriumalklylphosphate)

| | | |
|---|---|---|
| A) | Servoxyl VPDZ 6/100 (Sodium Isotrideceth-6 Phosphate) | 0,30 % |
| | Triethanolamin | 0,08 % |
| | Kokosfett + 150 EO | 0,63 % |
| | Glycerin | 2,98 % |
| | Demin. Wasser | 1,06 % |
| B) | MIGLYOL® 812 (Caprylic/Capric Triglyceride) | 7,07 % |
| | Octyl Stearate | 8,49 % |
| | Shea Butter | 4,71 % |
| | COSMACOL ECI (Tri-C12-C13 Alkyl Citrate) | 3,77 % |
| | COSMACOL EMI (Di-C12-C13 Alkyl Malate) | 3,77 % |
| | Fragrance Manderine & Neroli (Symrise) | 0,47 % |
| C) | Wasser (Aqua), Deionized | 66,70% |
| | Carbomer (Acrylates/C10-30 Alkyl Acrylate Crosspolymer) | 0,40 % |
| | NaOH | 0,20 % |
| | Phenonip | 1,00 % |

Herstellung: Zuerst wird die Phase C hergestellt. Hierbei wird das Carbomer unter Rühren vollständig gequollen. Phasen A und B werden jeweils homogen gemischt und auf 70°C erwärmt. Phase B wird unter Rühren im laminaren Strömungsfeld langsam der Phase A zugeführt. Nach Zugabe wird je nach Ansatzgröße unter Rühren (laminare Fließbedingungen müssen erhalten bleiben) kurz homogenisiert. Danach wird die Phase C eingerührt. Die Emulsion weist eine mittlere Tröpfchengröße von 286 nm auf.

### Beispiel 6: Emulsion für den Bereich Lebensmittel(-kontakt)

| | | |
|---|---|---|
| A) | ANIODAC AGCK-38 (Coco potassium soap, aqueous solution) | 3,4% |
| | IMWITOR 380 (Glyceryl Cocoate/Citrate/Lactate) | 0,6% |
| | Glycerin | 1,1% |
| B) | MIGLYOL® 812 (Caprylic/Capric Triglyceride) | 54,1% |
| C) | Wasser (Aqua), Deionized | 40,8% |

Herstellung: Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße von 400 nm auf.

### Beispiel 7: Kühlschmierstoffemulsion

| | | |
|---|---|---|
| A) | MARLOWET 4539 _{(Isononanol, ethoxyliert, propoxyliert, carboxymethyliert)} | 0,6% |
| | MARLOSOL FS _{(Ölsäure-polyethylenglykol-diester)} | 1,2% |
| | Glycerin | 1,2% |
| | Wasser (Aqua), Deionized | 1,8% |
| B) | Shell Gravex 915 | 46,3% |
| C) | Wasser (Aqua), Deionized | 48,9% |

Herstellung: Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße von 800 nm auf.

### Beispiel 8: Schalöl-Emulsion für die Bauchemie

| | | |
|---|---|---|
| A) | Servoxyl VPDZ 100 _{(C13-alcohol polyethylme glycol ether (6 EO) phosphate ester)} | 0,2% |
| | Triethanolamin | 0,1% |
| | PEG23 Laurylalkohol | 0,5% |
| | Wasser, deionized | 2,7% |
| B) | Schalöl | 20,0% |
| C) | Wasser, deionized | 76,5% |

Herstellung: Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Teilchengröße im Durchschnitt 280 nm.

### Beispiel 9: Siliconöl-Emulsion

| | | |
|---|---|---|
| A) | MARLINAT 242/90M_{(MIPA-Laureth Sulfate (and) Propylene Glycol)} | 115% |
| | COSMACOL ELI_{(C12-13 Alkyl Lactate)} | 0,7% |
| | Glycerin | 3,1% |
| | Wasser (Aqua), Deionized | 0,1% |
| B) | Wacker Silikonöl AK 300.000 | 51,0% |
| C) | Wasser (Aqua), Deionized | 43,4% |
| | Keltrol (Xanthan Gum) | 0,2% |

Herstellung: Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße von 900 nm auf.

### Beispiel 10: Siliconöl-Emulsion

| | | |
|---|---|---|
| A) | MARLINAT 242/90M _{(MIPA-Laureth Sulfate (and) Propylene Glycol)} | 1,5% |
| | MARLOWET COE 145 _{(Kokossölpolyalkylenglykolester)} | 0,7% |
| | Glycerin | 2,6% |
| | Wasser (Aqua), Deionized | 0,7% |
| B) | Wacker Silikonöl AK 300.000 | 61,1% |
| C) | Wasser (Aqua), Deionized | 33,3% |

Herstellung: Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße von 1400 nm auf.

### Beispiel 11: Siliconöl-Emulsion

| | | |
|---|---|---|
| A) | MARLINAT 242/90M _{(MIPA-Laureth Sulfate (end) Propylene Glycol)} | 2,1% |
| | SAFOL 23 _{(C12/13 Alkohol)} | 0,9% |
| | Glycerin | 3,4% |
| | Wasser (Aqua), Deionized | 1,0% |
| B) | Abil 350 (Polydimethylsiloxan) | 40,3% |
| C) | Wasser (Aqua), Deionized | 52,3% |

### Herstellung:

Phase A homogen mischen. Phase A vorlegen, Phase B unter Rühren im laminaren Strömungsfeld (ca. 1500 U/ min; Drahtrührer) langsam zudosieren, 1 min nachrühren. Phase C unter Rühren zugeben. Die Emulsion weist eine mittlere Tröpfchengröße von 200 nm auf.

## Patentansprüche

1. Verfahren zur Herstellung von Öl-in-Wasser-Dispersionen aus selbstemulgierenden Gelkonzentraten umfassend die folgenden Schritte:
(a) Bereitstellen eines Emulgatorkonzentrates (A) enthaltend zumindest
(A.1) zu 0,1 bis 75 Gew.% ein oder mehrere Polyole (P),
(A.2) zu 5 bis 80 Gew.% Wasser (W),
(A.3) zu 5 bis 40 Gew.% ein Ionisches Tensid (I) und/oder ein nichtionisches Tensid (N),
bezogen auf das Emulgatorkonzentrat (A),
(b) in Kontakt bringen einer Ölphase (O) mit dem Emulgatorkonzentrat (A) in einem im Wesentlichen ausschließlich laminaren Strömungsfeld, um ein selbstemulgierendes O/W Gelkonzentrat (G) mit einem Ölgehalt von 60 bis 99 Gew.% zu erhalten, und
(c) Zusammenbringen des Gelkonzentrates (G) mit Wasser und, das auch weitere Zuschlagstoffe enthalten kann,
wobei die Öl-in-Wasser-Dispersion eine Emulsion ist und die Ölphase (O) die disperse Phase ist.

2. Verfahren nach Anspruch 1, **dadurch gekennzeichnet, dass** das Emulgatorkonzentrat (A) unabhängig voneinander aufweist
(A.2) zu 50 bis 70 Gew.% Wasser (W),
(A.3) zu 10 bis 30 Gew.% ein ionisches Tensid (I) und/oder ein nichtionisches Tensid (N).

3. Verfahren nach Anspruch 1, **dadurch gekennzeichnet, dass** das O/W Gelkonzentrat (G) einen Ölgchalt von 80 bis 98 Gew.% aufweist.

4. Verfahren nach Anspruch 1, **dadurch gekennzeichnet, dass** das O/W Gelkonzentrat sich in Wasser selbsttätig ohne Einwirken von Scherkräften zum Erhalt einer Makroemulsion (M), im thermodynamischen Sinne, und insbesondere einer Nanoemulsion (C) einarbeitet.

5. Verfahren nach Anspruch 1, **dadurch gekennzeichnet, dass** das Wasser in Schritt (c) weniger als 5 Gew.%, vorzugsweise weniger als 2,5 Gew,%, Zuschlagstoffe enthält.

6. Verfahren nach Anspruch 1, **dadurch gekennzeichnet, dass** die Ölphase besteht aus oder enthält
- Triglycerinester von C8- bis C24-Carbonsäuren, insbesondere C12- bis C18-Carbonsäuren,
- Di-, Tri- oder Polyhydroxyverbindungen partiell oder vollständig verestert mit C6- bis C32-Carbonsäuren oder C6- bis C32-Hydroxycarbonsäuren,
- C12- bis C15-Alkylbenzoate,
- Di(C12-C32)ethcr,
- Ester (>C32) und/oder
- Silikonölen, insbesondere Polydimethylsiloxan.

7. Verfahren nach Anspruch 1, **dadurch gekennzeichnet, dass** das O/W Gelkonzentrat Verdicker enthält, insbesondere Cellulose, Cellulose Gum, Magnesium/Aluminiumsilikate und Organotonderivate, Kieselsäurederivate, Gummi Arabicum, Tragacanth Gum, Alginate und/oder Xanthan Gum.

8. Verfahren nach Anspruch 1, **dadurch gekennzeichnet, dass** das nichtionische Tensid ausgewählt ist aus einem oder mehreren Mitgliedern der Gruppe:
- C2- bis C4-Alkoxylate von linearen oder verzweigten C10- bis C22-Alkoholen, in statistischer Verteilung der Alkoxylat-Gruppen oder in Blockstruktur, vorzugsweise mit zumindest 6 Alkoxylat-Gruppen,
- Monoglycerinester von C8- bis C24-Carbonsäuren,
- C8- bis C32-Alkoxypolyglycoside,
- Poly(C2- bis C4-)alkylenglykol-Glyceryl-Fettssäureester,
- Ox(C2- bis C4-)alkylierte Sorbitan(C8- bis C32-)ester,
- C2- bis C4-Alkoxylate von Mono und Difett(>C8)säuren,
- Kondensationsprodukte von Ethylenoxid mit den Produkt aus der Reaktion von Propylenoxid und Ethylendiaminen,
- N,N'-Diacylalkylendiamin(C2- bis C4-)alkoxylate,
- ethoxylierte Fett(>C8)amine,
- (C2- bis C4-)alkoxylierte N-Acylamide, N-Acyl-N-Alkylamidalkoxylate,
- nichtionische Geminitenside,
- N-Methylgluconamide mit einem C6- bis C32-Kohlenwasserstoffrest,
- C2- bis C4-Alkoxylate von Fettsäure(>C8)-Triglyceriden und
- mit C8- bis C22-Fettsäuren teilveresterte Polyglycerine mit 3 bis 20 Glycerin-Einheiten, vorzugsweise 3 bis 10.

9. Verfahren nach Anspruch 1, **dadurch gekennzeichnet, dass** das ionische Tensid ausgewählt ist aus einem oder mehreren Mitgliedern der Gruppe:
- C8- bis C18-Alkylethersulfate,
- Sulfosuccinate in Kombination mit C6- bis C15-Fettalkoholen,
- Fettsäuren mit 8 bis 30 Kohlenstoffatomen und deren Seifen,
- Glycerin-Mono- und Diester von C8- bis C24-, insbesondere C12- bis C18-, Carbonsäuren,
- Carbonsäuren, die mit Milch- und Zitronen- oder Weinsäure verestert worden sind und ggf. ganz oder teilweise neutralisiert sind,
- Sorbitanester von C8- bis C32-, insbesondere C12- bis C18-, Alkancarbonsäuren, die mit Milch- und Zitronen- oder Weinsäure verestert worden sind und ggf. ganz oder teilweise neutralisiert sind,
- Mono-, Di- und Trialkylphosphorsäureester und deren C2- und C3-Alkoxylate und Mischvarianten,
- C10- bis C24-Olefinsulfonate,
- beta-C8- bis C18-Alkyloxyalkansulfonate,
- anionische Geminitenside,
- Aminoalkanoate der Formel R-NH(CH₂)ₙCOOM mit n = 1 bis 4, R = C8- bis C22-Alkyl oder Alkenyl und M = Wasserstoff, Alkalimetall, Erdalkalimetall, Ammonium oder Alkanolammonium,
- zwitterionische (Betain-)Tenside,
- Phosphate von C2- bis C4-alkoxylierten C8- bis C22-Alkoholen, insbesondere Tridecylalkohol,
- Phosphate linearer oder verzweigter C8- bis C22-Alkohole, und
- C2- bis C4-Alkoxylate von linearen oder verzweigten C8- bis C22-Alkoholen, in statistischer Verteilung der Alkoxylat-Gruppen oder in Blockstruktur, vorzugsweise mit zumindest 6 Alkoxylat-Gruppen, carboxymethyliert und verseift.

10. Verfahren nach Anspruch 1, **dadurch gekennzeichnet, dass** zumindest ein ionisches Tensid gemäß Anspruch 9 eingesetzt wird zusammen mit einem verzweigten Fettalkohol mit zumindest 8 Kohlenstoffatomen, insbesondere zumindest eines der nachgenannten ionischen Tenside:
- C8- bis C18-Alkylethersulfate,
- C10- bis C24-Olerinsulfonate,
- beta-C8- bis C18-Alkyloxyalkansulfonate,
- Phosphate von C2- bis C4-alkoxylierten C8- bis C22-Alkoholen, insbesondere Tridecylalkohol,
- Phosphate linearer oder verzweigter C8- bis C22-Alkohole und/oder
- C12- bis C15 lineare oder verzweigte Alkyllactate.

11. Verfahren nach Anspruch 1, **dadurch gekennzeichnet, dass** die Öl in Wasser Dispersion mindestens 50 bis 98 Gew.% Wasser und 1 bis 50 Gew.% Öl aufweist.

12. Verfahren nach Anspruch 1, **dadurch gekennzeichnet, dass** das Polyol Glycerin ist.

## Claims

1. A method for producing oil-in-water dispersions from self-emulsifying gel concentrates, comprising the following steps:
(a) providing an emulsifier concentrate (A) comprising at least:
(A.1) 0.1 to 75 % by weight of one more polyols (P);
(A.2) 5 to 80 % by weight of water (W); and
(A.3) 5 to 40 % by weight of an ionic surfactant (I) and/or a non-ionic surfactant (N);
each with respect to the emulsifier concentrate (A);
(b) bringing an oil phase (O) into contact with the emulsifier concentrate (A) in an essentially exclusively laminar flow field in order to obtain a selfemulsifying O/W gel concentrate (G) with an oil content of 66 to 99 % by weight; and
(c) bringing the O/W gel concentrate (G) into contact with water, which may optionally comprise further additives;
wherein the oil-in-water dispersion is an emulsion and the oil phase (O) is the disperse phase.

2. The method according to claim 1, **characterized in that** the emulsifier concentrate (A) comprises independently from each other:
(A.2) 50 to 70 % by weight of water (W),
(A.3) 10 to 30 % by weight, of an ionic surfactant (I) and/or a non-ionic surfactant (N).

3. The method according to claim 1, **characterized in that** the O/W gel concentrate (G) has an oil content of 80 to 98 % by weight.

4. The method according to claim 1, **characterized in that** the O/W gel concentrate self-actingly incorporates into water without the action of shear forces to obtain a macroemulsion (M), in the thermodynamic sense, and in particular into a nanoemulsion (C).

5. The method according to claim 1, **characterized in that** the water in step (c) contains less than 5% by weight, preferably less than 2.5% by weight, of additives.

6. The method according to claim 1, **characterized in that** the oil phase consists of or comprises:
• triglycerin esters of C8- to C24- carbonic acids, in particular C12- to C18-carbonic acids;
• di-, tri- or poly-hydroxy compounds partially or completely esterified with C6 to C32 carbonic acids or C6 to C32 hydroxycarbonic acids;
• C12-C15- alkylbenzoates;
• di-(C12-C32)ethers;
• esters (> C32); and/or
• silicone oils, in particular polydimethylsiloxane.

7. The method according to claim 1, **characterized in that** the O/W gel concentrate contains thickeners, in particular cellulose, cellulose gum, magnesium/aluminium silicates and organoclay derivatives, silica derivatives, gum arabic, tragacanth gum, alginates and/or xanthan gum.

8. The method according to claim 1, **characterized in that** the non-ionic surfactant is selected from one or more members of the following group:
• C2- to C4- alkoxylates of linear or branched ClO- to C22- alcohols, in a statistical distribution of the alkoxylate groups or in a block structure, preferably with at least 6 alkoxylate groups;
• mono-glycerin esters of C8- to C24- carbonic acids;
• C8- to C32 alkylpolyglycosides;
• poly(C2- to C4-)alkyleneglycol-glyceryl fatty acid esters;
• oxy(C2- to C4-)alkylated sorbitan(C8- to C32-)esters;
• C2- to C4- alkoxylates of mono- and dibasic fatty (> C8)acids;
• condensation products of ethylene oxide with the product from the reaction of propylene oxide and ethylenediamines;
• N,N'-Diacylalkylendiamine(C2- to C4-)alkoxylates;
• ethoxylated fatty (> C8)amines;
• (C2- to C4-)alkoxylated N-acylamide N-acyl-N-alkylamidalkoxylates;
• non-ionic gemini surfactants; and
• N-methylgluconamides with a C6- to C32- hydrocarbon residue;
• C2- to C4- alkoxylates of fatty acid(> C8) triglycerides; and
• polyglycerins with 3 to 20 glycerin units, preferably 3 to 10, partially esterified with C8- to C22-fatty acids.

9. The method according to claim 1, **characterized in that** the ionic surfactant is selected from one or more members of the following group:
• C8- to C 18- alkylethersulphates;
• sulphosuccinates in combination with C6- to C15-fatty alcohols;
• fatty acids with 8 to 30 carbon atoms and their soaps;
• glycerin mono- and di-esters of C8- to C24-, in particular C12- to C 18-carbonic acids;
• carbonic acids esterified with lactic and citric or tartaric acid and which if necessary have been completely or partially neutralized;
• sorbitan esters of C8- to C32-, in particular C12- to C18- alkancarbonic acids, esterified with lactic and citric or tartaric acid and if necessary have been completely or partially neutralized
• mono-, di- and tri-alkylphosphoric acid esters and their C2- and/or C3-alkoxylates and their mixtures;
• ClO- to C24- olefin sulphonates;
• beta-C8- to C18- alkyloxyalkanesulphonates;
• anionic gemini surfactants;
• aminoalkanoates with formula R-NH(CH₂)ₙCOOM where n, m = 1 to 4, R = C8-C22- alkyl or alkenyl and M = hydrogen, alkali metal, alkaline-earth metal, ammonium or alkanolammonium;
• zwitterionic (betaine-)surfactants;
• phosphates of C2- to C4- alkoxylated C8- to C22- alcohols, in particular tridecylalcohol;
• phosphates of linear or branched C8- to C22- alcohols; and
• C2- to C4- alkoxylates of linear or branched C8- to C22- alcohols, in a statistical distribution of the alkoxylate groups or in a block structure, preferably having at least 6 alkoxylate groups, carboxymethylated and in the soap form.

10. The method according to claim 1, **characterized in that** at least one ionic surfactant according to claim 9 is used together with a branched fatty alcohol containing at least 8 carbon atoms, in particular at least one of the following cited ionic surfactants:
• C8- to C18- alkylether sulphates;
• ClO- to C24- olefin sulphonates;
• beta-C8- to C18- alkyloxyalkanesulphonates;
• phosphates of C2- to C4- alkoxylated C8- to C22- alcohols, in particular tridecylalcohol;
• phosphates of linear or branched C8- to C22- alcohols; and/or
• C12- to C15- linear or branched alkyl lactates.

11. The method according to claim 1, **characterized in that** the oil-in-water dispersion has at least 50% to 98% by weight of water and 1% to 50% by weight of oil.

12. The method according to claim 1, **characterized in that** the polyol is glycerin.

## Revendications

1. Procédé de préparation de dispersions huile dans eau à partir de concentrés de gels auto-émulsifiants, comprenant les étapes suivantes consistant à :
(a) mettre à disposition un concentré émulsifiant (A) contenant au moins
(A.1) de 0,1 à 75 % en poids d'un ou de plusieurs polyols (P),
(A.2) de 5 à 80 % en poids d'eau (E) et
(A.3) de 5 à 40 % en poids d'un tensioactif ionique (I) et/ou d'un tensioactif non ionique (N)
par rapport au concentré émulsifiant (A),
(b) mettre en contact une phase huileuse (H) avec le concentré émulsifiant (A) dans un champ de flux sensiblement exclusivement laminaires pour obtenir un concentré de gels (G) auto-émulsifiant H/E ayant une teneur en huile allant de 60 à 99 % en poids, et
(c) réunir le concentré de gels (G) avec l'eau, qui peut contenir également d'autres additifs,
dans lequel la dispersion huile dans eau est une émulsion et la phase huileuse (H) est la phase dispersée.

2. Procédé selon la revendication 1, **caractérisé en ce que** le concentré émulsifiant (A) présente, indépendamment les uns des autres :
(A.2) de 50 à 70 % en poids d'eau (E),
(A.3) de 10 à 30 % en poids d'un tensioactif ionique (I) et/ou d'un tensioactif non ionique (N).

3. Procédé selon la revendication 1, **caractérisé en ce que** le concentré de gel (G) H/E présente une teneur en huile de 80 à 98 % en poids.

4. Procédé selon la revendication 1, **caractérisé en ce que** le concentré de gel H/E s'incorpore dans l'eau de manière autonome sans l'action de forces de cisaillement pour l'obtention d'une macroémulsion (M), au sens thermodynamique, et en particulier d'une nanoémulsion (C).

5. Procédé selon la revendication 1, **caractérisé en ce que** l'eau à l'étape (c) contient moins de 5 % en poids, de préférence moins de 2,5 % en poids d'additifs.

6. Procédé selon la revendication 1, **caractérisé en ce que** la phase huileuse se compose ou comprend
• des esters triglycérinés d'acides carboxyliques en C₈ à C₂₄, en particulier d'acides carboxyliques en C₁₂ à C₁₈,
• des composés di-, tri- ou polyhydroxy partiellement ou entièrement estérifiés avec des acides carboxyliques en C₆ à C₃₂ ou des acides hydroxycarboxyliques en C₆ à C₃₂,
• des alkyl(en C₁₂ à C₁₅)benzoates,
• des diéthers (en C₁₂ à C₃₂),
• des esters (>C₃₂) et/ou
• des huiles siliconées, en particulier du polydiméthylsiloxane.

7. Procédé selon la revendication 1, **caractérisé en ce que** le concentré de gel H/E comprend des épaississants, en particulier de la cellulose, de la gomme cellulosique, des silicates de magnésium/ d'aluminium et des dérivés d'organo-argile, des dérivés d'acide silicique, de la gomme arabique, de la gomme tragacanthe, des alginates et/ou de la gomme xanthane.

8. Procédé selon la revendication 1, **caractérisé en ce que** le tensioactif non ionique est choisi parmi un ou plusieurs membres du groupe constitué par :
• les alcoxylates en C₂ à C₄ d'alcools en C₁₀ à C₂₂ linéaires ou ramifiés, en distribution statistique des groupes alcoxylates ou en structure en blocs, ayant de préférence au moins 6 groupes alcoxylates,
• les esters monoglycérinés d'acides carboxyliques en C₈ à C₂₄,
• les alcoxy(en C₈ à C₃₂)polyglycosides,
• les esters d'acides gras glycérylés de polyalkylène(en C₂ à C₄)glycol,
• les esters (en C₈ à C₃₂) de sorbitane oxyalkylés (en C₂ à C₄),
• les alcoxylates en C₂ à C₄ de mono- et diacides gras (>C₈),
• les produits de condensation de l'oxyde d'éthylène et du produit de la réaction de l'oxyde de propylène et des éthylènediamines ;
• les alcoxylates (en C₂ à C₄) de la N,N'-diacylalkylènediamine,
• les amines grasses (> C₈) éthoxylées,
• les N-acylamides alcoxylés (en C₂ à C₄), les alcoxylates de N-acyl-N-alkylamide,
• les tensioactifs géminés non ioniques,
• les N-méthylgluconamides ayant un radical hydrocarbure en C₆ à C₃₂,
• les alcoxylates en C₂ à C₄ de triglycérides d'acides gras (>C₈) et
• les polyglycérines partiellement estérifiées avec des acides gras en C₈ à C₂₂ ayant de 3 à 20 motifs de glycérine, de préférence 3 à 10.

9. Procédé selon la revendication 1, **caractérisé en ce que** le tensioactif ionique est choisi parmi un ou plusieurs membres du groupe constitué par :
• les alkyl(en C₈ à C₁₈) éther-sulfates,
• les sulfosuccinates en combinaison avec des alcools gras en C₆ à C₁₅,
• les acides gras ayant de 8 à 30 atomes de carbone et leurs savons,
• les mono- et diesters glycérinés d'acides carboxyliques en C₈ à C₂₄, en particulier en C₁₂ à C₁₈,
• les acides carboxyliques qui ont été estérifiés avec de l'acide lactique et citrique ou tartrique et ont été le cas échéant entièrement ou partiellement neutralisés,
• les esters de sorbitane d'acides alcanecarboxyliques en C₈ à C₃₂, en particulier en C₁₂ à C₁₈, qui ont été estérifiés avec de l'acide lactique et citrique ou tartrique et ont été le cas échéant entièrement ou partiellement neutralisés,
• les esters d'acides mono-, di- et trialkylphosphoriques et leurs alcoxylates en C₂ et C₃ et variantes mixtes,
• les sulfonates d'oléfine en C₁₀ à C₂₄,
• les bêta-alkyloxy(en C₈ à C₁₈)alcanesulfonates,
• les tensioactifs géminés anioniques,
• les aminoalcanoates de formule R-NH(CH₂)ₙCOOM avec n = 1 à 4, R = groupe alkyle en C₈ à C₂₂ ou alcényle et M = hydrogène, métal alcalin, métal alcalinoterreux, ammonium ou alcanolammonium,
• les tensioactifs (de bétaïne) zwitterioniques,
• les phosphates d'alcools en C₈ à C₂₂ alcoxylés en C₂ à C₄, en particulier l'alcool tridécylique,
• les phosphates d'alcools en C₈ à C₂₂ linéaires ou ramifiés, et
• les alcoxylates en C₂ à C₄ d'alcools en C₈ à C₂₂ linéaires ou ramifiés, en distribution statistique des groupes alcoxylates ou en structure en blocs, ayant de préférence au moins 6 groupes alcoxylates, carboxyméthylés et saponifiés.

10. Procédé selon la revendication 1, **caractérisé en ce qu'**au moins un tensioactif ionique selon la revendication 9 est mis en oeuvre conjointement avec un alcool gras ramifié comprenant au moins 8 atomes de carbone, en particulier conjointement avec au moins un des tensioactifs ioniques cités ci-après :
• les alkyl(en C₈ à C₁₈) éther-sulfates,
• les sulfonates d'oléfine en C₁₀ à C₂₄,
• les bêta-alkyloxy(en C₈ à C₁₈)alcanesulfonates,
• les phosphates d'alcools en C₈ à C₂₂ alcoxylés en C₂ à C₄, en particulier l'alcool tridécylique,
• les phosphates d'alcools en C₈ à C₂₂ linéaires ou ramifiés et/ou
• les alkyl(en C₁₂ à C₁₅)lactates linéaires ou ramifiés.

11. Procédé selon la revendication 1, **caractérisé en ce que** la dispersion huile dans eau présente au moins 50 à 98 % en poids d'eau et 1 à 50 % en poids d'huile.

12. Procédé selon la revendication 1, **caractérisé en ce que** le polyol est la glycérine.
